(19) Europäisches Patentamt
European Patent Office
Office européen des brevets

(11) Veröffentlichungsnummer : **0 638 597 A1**

(12) # EUROPÄISCHE PATENTANMELDUNG

(21) Anmeldenummer : **94810398.1**

(22) Anmeldetag : **05.07.94**

(51) Int. Cl.[6] : **C08F 20/36,** C08F 22/22, C08F 22/40, C08F 12/22, C07D 211/06, C07D 211/76, C07D 241/08, C07D 471/10, C07D 491/10, C07D 498/10, C09D 133/04

(30) Priorität : **13.07.93 CH 2098/93**

(43) Veröffentlichungstag der Anmeldung : **15.02.95 Patentblatt 95/07**

(84) Benannte Vertragsstaaten : **BE DE ES FR GB IT NL**

(71) Anmelder : **CIBA-GEIGY AG**
**Klybeckstrasse 141**
**CH-4002 Basel (CH)**

(72) Erfinder : **Steinmann, Alfred, Dr.**
**Les Russilles**
**CH-1724 Praroman (CH)**

(54) **Polymere Addukte von gehinderten Amin-Epoxiden als Stabilisatoren.**

(57) Es werden Verbindungen beschrieben, welche durch Polymerisation von Estern oder Phenolethern der Formel V

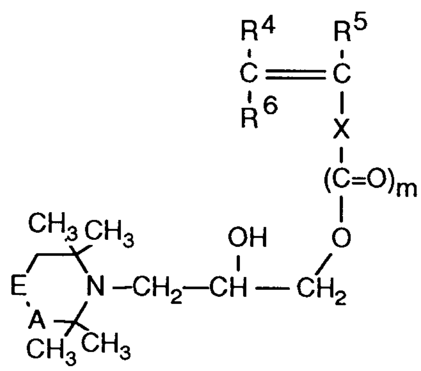

(V)

und/oder der Formel VI

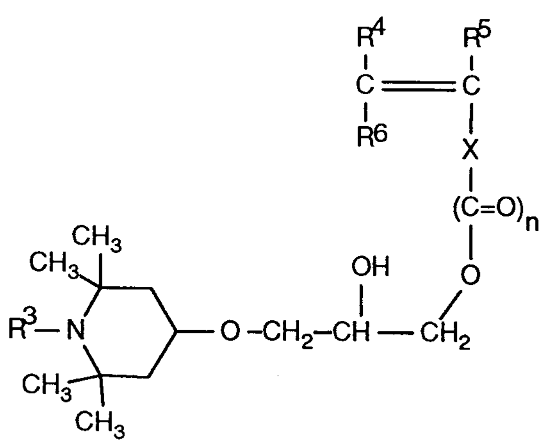

(VI)

worin m und n jeweils 0 oder 1 sind und A, E, $R^1$ bis $R^6$ und X die in Anspruch 1 angegebenen Bedeutungen umfassen,

EP 0 638 597 A1

und bis zu 50 Mol-% anderer, ethylenisch ungesättigter Verbindungen erhältlich sind. Die erfindungsgemäßen Homo- oder Copolymere lassen sich vorteilhaft zur Stabilisierung organischer Polymere gegen den schädigenden Einfluß von Licht, Sauerstoff und/oder Wärme einsetzen.

Die Erfindung betrifft neue Verbindungen, die durch radikalische Polymerisation ethylenisch ungesättigter 3-(2,2,6,6-Tetramethyl-piperidin-4-yl-oxy)- oder 3-(2,2,6,6-Tetramethyl-piperidin-1-yl)-2-hydroxy-propyl-carboxylate beziehungsweise entsprechender Vinylphenolether erhalten werden können, deren Verwendung als Stabilisatoren organischen Materials gegen den schädigenden Einfluß von Licht, Sauerstoff und/oder Wärme, neue monomere Ausgangsprodukte, sowie die entsprechenden stabilisierten Zusammensetzungen.

Die Herstellung einiger Verbindungen vom Typ des 2,2,6,6-Tetramethyl-4-(2,3-epoxypropoxy)-piperidin sowie ihre Verwendung als Stabilisatoren für organische Polymere wird beispielsweise durch Luston und Vass, Makromol. Chem., Macromol. Symp. 27, 231 (1989), beschrieben.

Die Bindung derartiger reaktiver Polyalkylpiperidine an Fluorpolymere, die freie Carboxygruppen enthalten, wird in EP-A-526 399 beschrieben.

Die Publikation EP-A-001835 lehrt die weitere Umsetzung der Epoxygruppen-haltigen Piperidine mit Dicarbonsäureanhydriden zu Polyestern.

Es besteht weiterhin Bedarf an neuen polymeren Lichtschutzmitteln mit verbesserten Gebrauchseigenschaften, welche Tetramethylpiperidin-Gruppen als Seitenketten enthalten.

Gegenstand der Erfindung sind daher zunächst Polymere mit Tetramethylpiperidin-Seitenketten enthaltend 50 bis 100 Mol-% strukturelle Einheiten der Formel I und/oder II

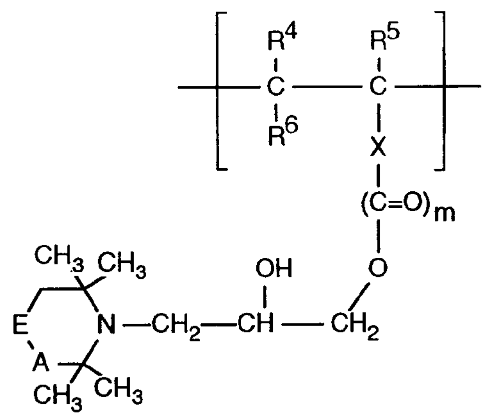

(I)

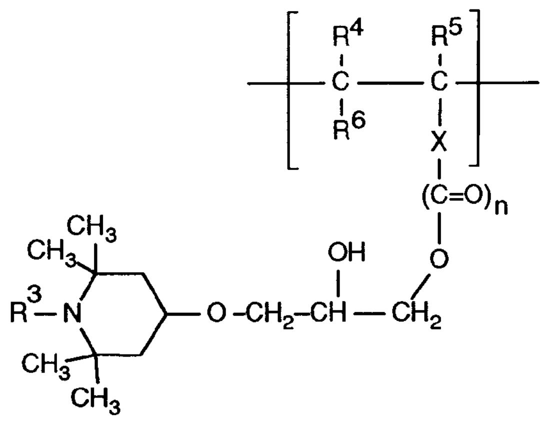

(II)

wobei das mittels Gelpermeationschromatographie gemessene Molekulargewicht $M_n$ des Homo- oder Copolymers zwischen 600 und 600 000 g/Mol beträgt, und
wobei das unten definierte k die Zahl 2 oder 3 ist;
m und n, unabhängig voneinander, 0 oder 1 darstellen;
A $-CH_2-$ oder -CO- bedeutet;
wenn A Methylen ist, E die Bedeutung

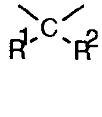

hat, und
wenn A Carbonyl ist, E die Bedeutung >N-$R^{17}$ hat;
$R^1$ Wasserstoff;
$R^2$ Wasserstoff; -N($R^{13}$)$R^{14}$; oder $C_1$-$C_{50}$-Alkoxy; oder $C_2$-$C_{50}$-Alkoxy bedeutet, welches durch -O-, -S-,-CO-N($R^{17}$)-, -N($R^{17}$)-CO- oder -N$R^{11}$- und/oder durch $C_5$-$C_8$-Cycloalkylen oder Phenylen unterbrochen ist und/oder welches 1 bis 3 tertiäre Hydroxylgruppen enthält; oder $R^2$ $C_5$-$C_{12}$-Cycloalkoxy; durch 1 bis 4 Reste -$R^{12}$ substituiertes $C_5$-$C_{12}$-Cycloalkoxy; Phenoxy; durch 1 bis 4 -$R^{12}$ substituiertes Phenoxy; oder $C_7$-$C_{20}$-Aralkoxy bedeutet; oder
$R^1$ und $R^2$ gemeinsam einen Substituenten =O bedeuten; oder zusammen mit dem Kohlenstoffatom, an das sie gebunden sind, einen Fünf- oder Sechsring einer der Formeln

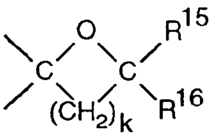 , 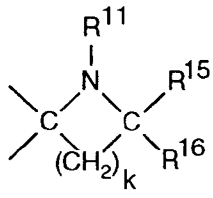 , 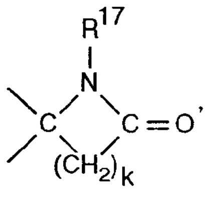 , 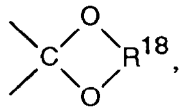 , 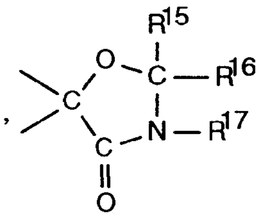 ,

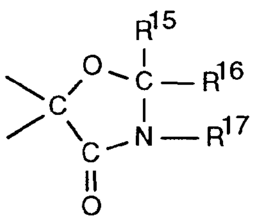 , 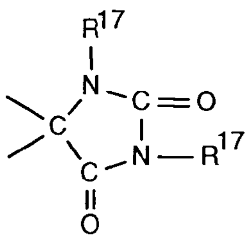

darstellen;
$R^3$ Wasserstoff; $C_1$-$C_{36}$-Alkyl, $C_1$-$C_{36}$-Alkoxy, $C_7$-$C_{36}$-Aralkyl oder $C_7$-$C_{36}$-Aralkoxy, die jeweils unsubstituiert oder durch $C_5$-$C_8$-Cycloalkyl oder -CO-N($R^{17}$)$_2$ substituiert oder im aliphatischen Teil durch $C_5$-$C_8$-Cycloalkylen -CO-N($R^{17}$)- oder -N($R^{17}$)-CO- oder Sauerstoff oder Schwefel unterbrochen oder im aromatischen Teil durch 1 bis 3 $C_1$-$C_4$-Alkyl- oder $C_1$-$C_4$-Alkoxyreste substituiert sind; $C_5$-$C_{12}$-Cycloalkyl, welches unsubstituiert oder durch 1 bis 3 $C_1$-$C_4$-Alkyl- oder $C_1$-$C_4$-Alkoxyreste substituiert ist; $C_5$-$C_{12}$-Cycloalkoxy, welches unsubstituiert oder durch 1 bis 3 $C_1$-$C_4$-Alkyl- oder $C_1$-$C_4$-Alkoxyreste substituiert ist; $C_6$-$C_{10}$-Aryl, welches unsubstituiert oder durch 1 bis 3 $C_1$-$C_4$-Alkyl- oder $C_1$-$C_4$-Alkoxyreste substituiert ist; $C_6$-$C_{10}$-Aryloxy, welches unsubstituiert oder durch 1 bis 3 $C_1$-$C_4$-Alkyl- oder $C_1$-$C_4$-Alkoxyreste substituiert ist; -CO-$R^{11}$; oder Benzoyl oder Naphtoyl, welche jeweils durch $C_1$-$C_4$-Alkyl substituiert sind, bedeutet;
$R^4$ und $R^5$, unabhängig voneinander, Wasserstoff; $C_1$-$C_{18}$-Alkyl; Phenyl; oder $C_7$-$C_9$-Phenylalkyl sind; oder einer der $R^4$ oder $R^5$ zusätzlich Chlor umfaßt;
$R^6$ eine der für $R^4$ und $R^5$ angegebenen Bedeutungen außer Chlor hat; oder eine direkte Bindung zur Gruppe X bedeutet; oder eine Gruppe einer der Formeln -X-(CO)$_m$-$Z^4$ oder -X-(CO)$_n$-$Z^5$ darstellt;
$R^{11}$ $C_1$-$C_{18}$-Alkyl; $C_5$-$C_7$-Cycloalkyl; Phenyl; Naphthyl; $C_7$-$C_9$-Phenylalkyl; oder $C_{11}$-$C_{14}$-Naphthylalkyl;
$R^{12}$ $C_1$-$C_{18}$-Alkyl; $C_5$-$C_7$-Cycloalkyl; Phenyl oder Benzyl ist;
$R^{13}$ und $R^{14}$, unabhängig voneinander, $C_1$-$C_{50}$-Alkyl; oder $C_2$-$C_{50}$-Alkyl bedeuten, welches durch -O-, -S-, -CO-N($R^{17}$)-, -N($R^{17}$)-CO- oder -N$R^{11}$- und/oder durch $C_5$-$C_8$-Cycloalkylen oder Phenylen unterbrochen ist und/oder welches 1 bis 3 tertiäre Hydroxylgruppen enthält; oder $C_5$-$C_{12}$-Cycloalkyl, welches unsubstituiert oder durch 1 bis 4 Reste -$R^{12}$ substituiert ist; Phenyl, welches unsubstituiert oder durch 1 bis 4 -$R^{12}$ substituiert ist; oder $C_7$-$C_{20}$-Aralkyl bedeuten; oder
$R^{13}$ und $R^{14}$ zusammen mit dem Stickstoffatom, an das sie gebunden sind, ein zyklisches Imid der Formel

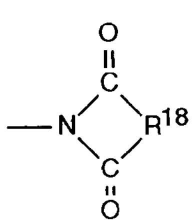

darstellen, dessen Ringstruktur 4 bis 6 Kohlenstoffatome enthält;
$R^{15}$ und $R^{16}$, unabhängig voneinander, H; oder $C_1$-$C_{12}$-Alkyl; oder gemeinsam geradkettiges, $\alpha,\omega$-verknüpftes $C_4$-$C_{13}$-Alkylen bedeuten;
$R^{17}$ Wasserstoff ist oder eine der Bedeutungen von $R^{11}$ hat;
$R^{18}$ $C_2$-$C_{18}$-Alkylen darstellt;
X im Fall, daß $R^6$ eine direkte Bindung zur Gruppe X darstellt, die trivalente Gruppe

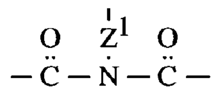

ist, wobei die an den beiden Carbonylgruppen lokalisierten freien Valenzen an die benachbarten Kohlenstoffatome der Polymerkette binden;
und X, wenn $R^6$ keine direkte Bindung zur Gruppe X darstellt, im Fall m = 0 beziehungsweise n = 0 Phenylen oder durch -$Z^4$ oder -$Z^5$ substituiertes Phenylen ist;
und X, wenn $R^6$ keine direkte Bindung zur Gruppe X darstellt, im Fall m = 1 beziehungsweise n = 1 eine direkte Bindung; $C_1$-$C_4$-Alkylen; Phenylen; oder $Z^2$-$Z^3$- bedeutet, wobei $Z^2$ an das Kohlenstoffatom der Polymerkette bindet;
$Z^1$ Phenylen;
$Z^2$ -O- oder Phenylen;
$Z^3$ $C_1$-$C_8$-Alkylen;
$Z^4$ eine Gruppe der Formel Ia

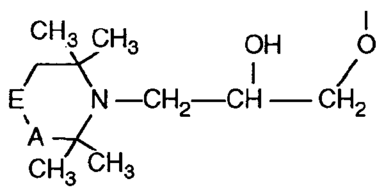

(Ia)

und $Z^5$ eine Gruppe der Formel IIa

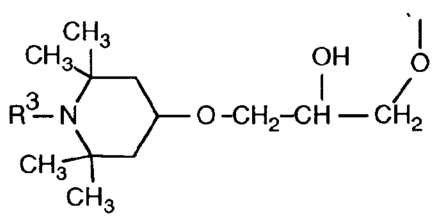

(IIa)

darstellt.

Unter einer tertiären Hydroxylgruppe ist ein Substituent des Typs -OH an einem tertiären Kohlenstoffatom zu verstehen. Dieses zeichnet sich dadurch aus, daß es mit seinen übrigen drei freien Valenzen an drei weitere Kohlenstoffatome bindet; die resultierenden Verbindungen werden auch als tertiäre Alkohole bezeichnet (vergl. Beyer/Walter, Lehrbuch der Organischen Chemie, 20. Auflage, S.59 und 111, S. Hirzel Verlag, Stuttgart 1984). Ein Rest, welcher eine tertiäre Hydroxylgruppe enthält, enthält daher mindestens 4 Kohlenstoffatome. Aryl bedeutet einen aromatischen Kohlenwasserstoffrest wie beispielsweise Phenyl oder Naphthyl. Aralkyl bedeutet Alkyl, das durch einen aromatischen Kohlenwasserstoffrest, z.B. einen $C_6$-$C_{10}$-Kohlenwasserstoffrest, substituiert ist; Beispiele dafür sind neben anderen Benzyl oder $\alpha$-Methylbenzyl.

Enthalten $R^2$, $R^3$, $R^{13}$ oder $R^{14}$ im Rahmen ihrer angegebenen Bedeutungen Alkyl oder Alkylen, welches durch -O-, -S-, -CO-N($R^{17}$)-, -N($R^{17}$)CO- oder -N($R^{11}$)- unterbrochen ist, so handelt es sich um Alkyl beziehungsweise Alkylen mit mindestens 2, vorzugsweise mindestens 4 Kohlenstoffatomen, das bevorzugt durch 1-6 Gruppen -O- oder -S-, besonders durch 1-6-O- unterbrochen ist; die Heteroatome oder Carbonylgruppen binden vorzugsweise an gesättigte Kohlenstoffatome und nicht an andere Heteroatome oder Carbonylgruppen, Peroxo- oder Hydrazinstrukturen treten in der Regel nicht auf. Besonders bevorzugt stellen diese Reste Polyoxyethylenketten dar, deren Enden mit $C_1$-$C_8$-Alkyl abgesättigt sind.

$R_2$ ist besonders bevorzugt ein langkettiger Rest oder Teil eines solchen, beispielsweise ein Rest enthaltend 5 bis 50 Kohlenstoffatome, insbesondere 8 bis 30 Kohlenstoffatome.

In den obigen Formeln von Substituenten, z.B. den Formeln Ia und IIa, sind freie Valenzen durch Striche dargestellt. Beispielsweise gibt die Teilformel

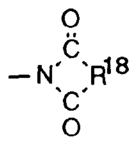

eine Bedeutungsmöglichkeit des Substituenten $R^2$ wieder, der waagerechte Strich stellt die Bindung an das Kohlenstoffatom in 4-Stellung im Piperidinring der Formel I oder Ia dar; die Teilformel

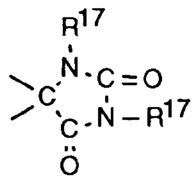

bezeichnet eine gemeinsame Bedeutungsmöglichkeit für die Reste $R^1$ und $R^2$, wobei die Striche Bindungen innerhalb des Piperidinringes der Formel I oder Ia darstellen, und zwar die Bindungen zwischen den Kohlenstoffatomen in 3- und in 4-, sowie zwischen den Kohlenstoffatomen in 4- und in 5-Stellung im Piperidinring.

Zwei gleichartige Reste in derselben Formel können dabei gleiche oder verschiedene Bedeutungen haben; beispielsweise kann einer der beiden Reste $R^{17}$ in der Teilformel $-CO-N(R^{17})_2$ Wasserstoff bedeuten, während der andere Rest $R^{17}$ eine der Bedeutungen von $R^{11}$ hat.

Erfindungsgemäße Polymere können beispielsweise folgende wiederkehrende Einheiten enthalten:

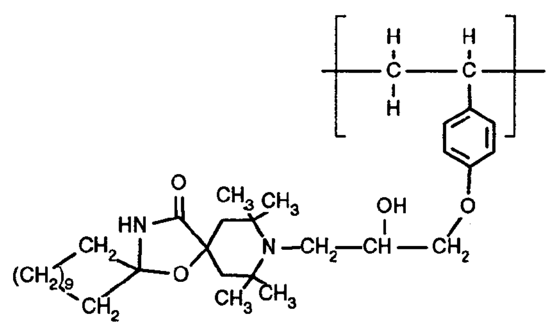

;

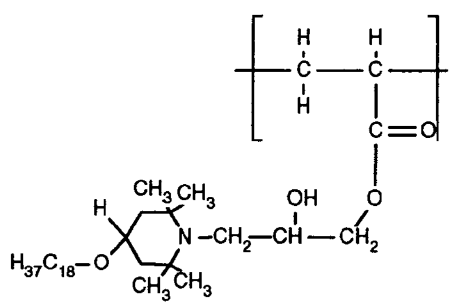

;

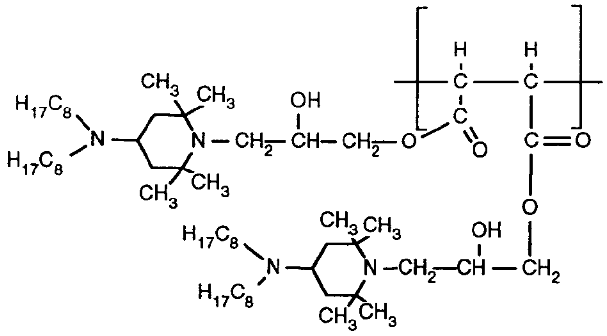

;

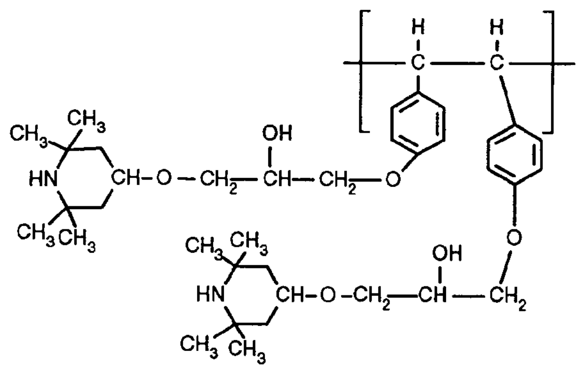

;

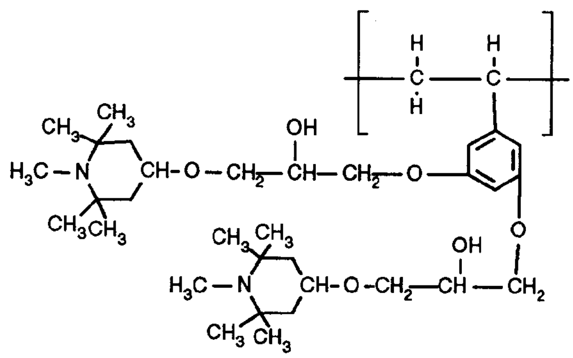

;

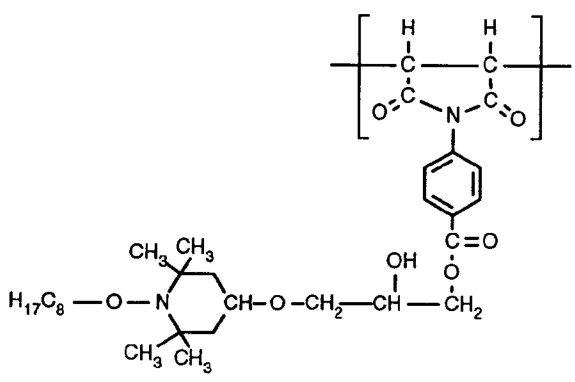

.

Die genannten Homo- oder Copolymere lassen sich vorteilhaft zur Stabilisierung organischer Polymere gegen den schädigenden Einfluß von Licht, Sauerstoff und/oder Wärme einsetzen.

Bedeuten die Reste $R^3$, $R^4$, $R^5$, $R^6$, $R^{11}$, $R^{12}$, $R^{13}$, $R^{14}$, $R^{15}$, $R^{16}$ und $R^{17}$ sowie die weiter unten aufgeführten

$R^7$, $R^8$, $R^9$ und $R^{10}$ Alkyl, so kann dies, im Rahmen der angegebenen Definitionen, verzweigtes oder unverzweigtes Alkyl sein wie Methyl, Ethyl, Propyl, Isopropyl, n-Butyl, sec-Butyl, Isobutyl, t-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl, Docosyl, Pentakosyl oder Triakosyl.

Beispiele für die Bedeutungen von $R^3$ sind unter anderen die folgenden:

Verzweigtes oder unverzweigtes $C_1$-$C_{36}$-Alkyl wie Methyl, Ethyl, Propyl, Isopropyl, n-Butyl, sec-Butyl, Isobutyl, t-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl, Docosyl, Pentakosyl oder Triakosyl; bevorzugt ist unverzweigtes Alkyl, besonders bevorzugt unverzweigtes $C_1$-$C_{18}$-Alkyl; verzweigtes oder unverzweigtes $C_1$-$C_{36}$-Alkyloxy, insbesondere $C_6$-$C_{18}$-Alkyloxy wie Hexyloxy, Heptyloxy, Octyloxy, Nonyloxy, Decyloxy, Undecyloxy, Dodecyloxy;

durch $C_5$-$C_8$-Cycloalkyl substituiertes Alkyl oder Alkyloxy wie Cyclopentylmethyl, Cyclohexylmethyl, Cycloheptylmethyl, Cyclooctylmethyl, Cyclohexylethyl, 2-Cyclohexyl-n-propyl, 3-Cyclohexyl-n-propyl, 4-Cyclohexyl-n-butyl;

durch $C_5$-$C_8$-Cycloalkylen oder 1 bis 6 -O- unterbrochenes Alkyl oder Alkyloxy wie beispielsweise der Formeln

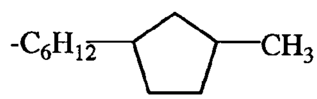 ,

$-C_2H_4$-O-$C_2H_4$-O-$C_{12}H_{25}$, $-(C_2H_4\text{-O})_4$-$C_4H_9$, $-(C_2H_4\text{-O})_6$-$C_4H_9$;

$C_5$-$C_8$-Cycloalkyl oder $C_5$-$C_8$-Cycloalkyloxy, das unsubstituiert oder mit Alkyl substituiert ist, wie Cyclopentyl, Cyclopentyloxy, Cyclohexyl, Cyclohexyloxy, Cycloheptyl, Cycloheptyloxy, Cyclooctyl, Cyclooctyloxy, 2- oder 4-Methylcyclohexyloxy, Dimethylcyclohexyloxy, Trimethylcyclohexyl, t-Butylcyclohexyl, besonders Cyclohexyl und Cyclohexyloxy;

Phenyl, Phenoxy; mit $C_1$-$C_4$-Alkyl substituiertes Phenyl oder Phenyloxy;

$C_7$-$C_{12}$-Phenylalkyl oder $C_7$-$C_{12}$-Phenylalkyloxy, wie z.B. Benzyl, Benzyloxy, Phenethyloxy, 3-Phenylpropyloxy, $\alpha$-Methylbenzyl, $\alpha$-Methylbenzyloxy, $\alpha,\alpha$-Dimethylbenzyl, $\alpha,\alpha$-Dimethylbenzyloxy.

Bevorzugt ist A Methylen und E eine Gruppe der Formel

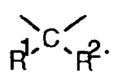

Ein bevorzugter Gegenstand der Erfindung ist ein Homo- oder Copolymer enthaltend 50 bis 100 Mol-% Struktureinheiten der Formel I und/oder II, sowie 0 bis 50 Mol-% Struktureinheiten der Formel III

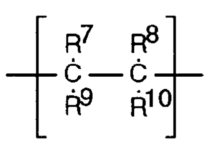 , (III)

dessen mittels Gelpermeationschromatographie gemessenes Molekulargewicht $M_n$ zwischen 1000 und 300 000 g/Mol beträgt, und worin

$R^7$, $R^8$ und $R^9$, unabhängig voneinander, Wasserstoff; -Cl; $C_1$-$C_{18}$-Alkyl; Phenyl; Phenyl, welches durch 1 bis 3 -Cl, $C_1$-$C_4$-Alkyl und/oder $C_1$-$C_4$-Alkoxy substituiert ist; oder $C_7$-$C_9$-Phenylalkyl sind;

$R^{10}$ eine der für $R^7$, $R^8$ und $R^9$ angegebenen Bedeutungen hat; oder -CN;

$C_1$-$C_{12}$-Alkyloxycarbonyl; $C_1$-$C_{12}$-Alkanoyloxy; $C_1$-$C_{12}$-Alkoxy; oder eine Gruppe der Formel IVa oder IVb ist

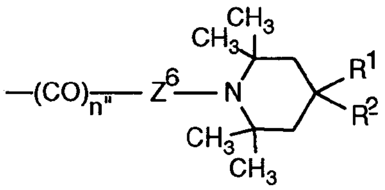

(IVa)

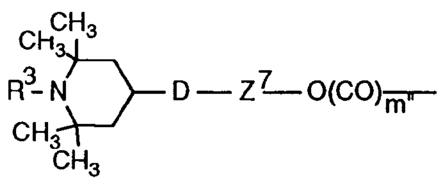

(IVb)

worin m" und n" jeweils die Zahl 0 oder die Zahl 1 darstellen;
D -O- oder $-NR^{11}-$ ist;
$R^1$, $R^2$, $R^3$ und $R^{11}$ die oben angegebenen Bedeutungen haben;
$Z^6$ eine direkte Bindung, $C_1$-$C_8$-Alkylen oder Polyoxyethylen gemäß der Formel $-CH_2-CH_2-(O-CH_2-CH_2)_h-$ darstellt, wobei h eine ganze Zahl aus dem Bereich 1 bis 30 ist; und
$Z^7$ $C_1$-$C_8$-Alkylen oder Polyoxyethylen gemäß der Formel $-CH_2-CH_2-(O-CH_2-CH_2)_h-$ darstellt, wobei h eine ganze Zahl aus dem Bereich 1 bis 30 ist.

Bei den oben und im folgenden dargestellten strukturellen Einheiten der Formeln I, II und III handelt es sich allgemein um konstitutionelle Repetiereinheiten (repeating units). Bei Copolymeren kann es sich beispielsweise um statistische, alternierende oder Blockcopolymerisate handeln.

Bevorzugt sind Polymere bestehend aus wiederkehrenden Einheiten der Formel I oder II, oder entsprechende Copolymere, die zwischen 50 und 100 Mol-% wiederkehrende Einheiten der Formel I und/oder der Formel II enthalten. Polmere bestehend aus wiederkehrenden Einheiten der Formel I oder II sind Homopolymere oder Copolymere, die aus 2 oder mehr unterschiedlichen Einheiten der Formel I oder II aufgebaut sind; besonders bevorzugt sind Homopolymere.

Unter den Copolymeren, die zwischen 50 und 100 Mol-% wiederkehrende Einheiten der Formel I und/oder der Formel II enthalten, sind diejenigen besonders bevorzugt, deren übrige strukturelle Einheiten der Formel III entsprechen, insbesondere diejenigen, die aus Einheiten der Formel I und III, oder aus Einheiten der Formel II und III aufgebaut sind (Typen A und B der nachstehenden Tabelle).
Einige zweckmäßige Zusammensetzungen der erfindungsgemäßen Copolymere, die aus Einheiten der Formeln I und/oder II und III aufgebaut sind, können der folgenden Tabelle entnommen werden:

| Typ | Formel I | Formel II | restliche Einheiten |
|---|---|---|---|
| A | 50-95 Mol-% | keine | Formel III |
| B | keine | 50-95 Mol-% | Formel III |
| C | 1-30 Mol-% | 1-30 Mol-% | Formel III |
| D | 10-60 Mol-% | 10-60 Mol-% | Formel III |
| E | 40-80 Mol-% | 1-30 Mol-% | Formel III |
| F | 1-30 Mol-% | 40-80 Mol-% | Formel III |

Vorzugsweise beträgt das Molekulargewicht $M_n$ des Homopolymeren aus Struktureinheiten der Formel I 1000 bis 300 000 g/Mol, das Molekulargewicht $M_n$ des Homopolymeren aus Struktureinheiten der Formel II 1000 bis 50 000 g/Mol und das Molekulargewicht $M_n$ des Copolymeren 1000 bis 100 000 g/Mol.
Besonders vorteilhaft lassen sich Homopolymere aus Struktureinheiten der Formel I einsetzen, deren Molekulargewicht $M_n$ 10 000 bis 300 000 g/Mol, oder Homopolymere aus Struktureinheiten der Formel II, deren Molekulargewicht $M_n$ 1000 bis 30 000 g/Mol, vor allem 1000 bis 10 000 g/Mol, oder die genannten Copolymere, deren Molekulargewicht $M_n$ 3000 bis 100 000 g/Mol beträgt.

Von besonderem Interesse ist ein Homo- oder Copolymer enthaltend 50 bis 100 Mol-% Struktureinheiten der Formel I und/oder II, worin
A Methylen und E eine Gruppe der Formel

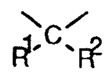

ist;
$R^1$ Wasserstoff;
$R^2$ Wasserstoff; $-N(R^{13})R^{14}$; $C_1$-$C_{18}$-Alkoxy; $C_4$-$C_{36}$-Alkoxy, welches durch $-CO-N(R^{17})-$ oder $-N(R^{17})-CO-$ unterbrochen ist; $C_5$-$C_8$-Cycloalkoxy, welches unsubstituiert oder durch $-R^{12}$ substituiert ist; Phenoxy, welches unsubstituiert oder durch 1 bis 4 $-R^{12}$ substituiert ist; oder $C_7$-$C_{12}$-Aralkoxy bedeutet; oder
$R^1$ und $R^2$ gemeinsam einen Substituenten =O bedeuten oder zusammen mit dem Kohlenstoffatom, an das sie gebunden sind, einen Fünf- oder Sechsring einer der Formeln

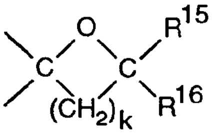 , 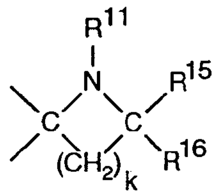 , 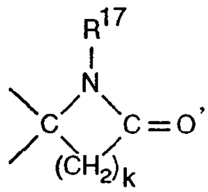 , 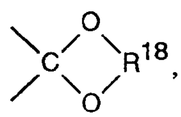 , 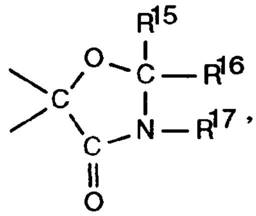 ,

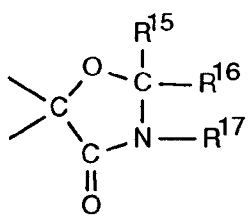 , 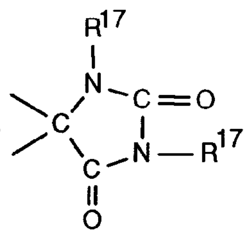

darstellen;
$R^3$ die Bedeutung $C_1$-$C_{36}$-Alkyl; $C_1$-$C_{36}$-Alkoxy; $C_5$-$C_{12}$-Cycloalkyl, welches unsubstituiert oder durch 1 bis 3 $C_1$-$C_4$-Alkyl- oder $C_1$-$C_4$-Alkoxyreste substituiert ist;
$C_5$-$C_{12}$-Cycloalkoxy, welches unsubstituiert oder durch 1 bis 3 $C_1$-$C_4$-Alkyl- oder $C_1$-$C_4$-Alkoxyreste substituiert ist; Phenyl, welches unsubstituiert oder durch 1 bis 3 $C_1$-$C_4$-Alkyl- oder $C_1$-$C_4$-Alkoxyreste substituiert ist; Naphthyl, welches unsubstituiert oder durch 1 bis 3 $C_1$-$C_4$-Alkyl- oder $C_1$-$C_4$-Alkoxyreste substituiert ist; Phenoxy, welches unsubstituiert oder durch 1 bis 3 $C_1$-$C_4$-Alkyl- oder $C_1$-$C_4$-Alkoxyreste substituiert ist; Naphthoxy, welches unsubstituiert oder durch 1 bis 3 $C_1$-$C_4$-Alkyl- oder $C_1$-$C_4$-Alkoxyreste substituiert ist; $C_7$-$C_{12}$-Phenylalkyl, welches unsubstituiert oder am Phenylring durch 1 bis 3 $C_1$-$C_4$-Alkyl- oder $C_1$-$C_4$-Alkoxyreste substituiert ist; $C_7$-$C_{12}$-Phenylalkoxy, welches unsubstituiert oder am Phenylring durch 1 bis 3 $C_1$-$C_4$-Alkyl- oder $C_1$-$C_4$-Alkoxyreste substituiert ist; $-CO-R^{11}$; oder Benzoyl oder Naphtoyl, welche jeweils durch $C_1$-$C_4$-Alkyl substituiert sind, hat;
$R^4$, $R^5$ und $R^6$, unabhängig voneinander, Wasserstoff; $C_1$-$C_{18}$-Alkyl; Phenyl; oder $C_7$-$C_9$-Phenylalkyl sind; oder einer der $R^4$ oder $R^5$ zusätzlich -Cl umfaßt;
$R^{11}$ $C_1$-$C_{18}$-Alkyl; $C_5$-$C_7$-Cycloalkyl; Phenyl; oder $C_7$-$C_9$-Phenylalkyl;
$R^{12}$ $C_1$-$C_{18}$-Alkyl; $C_5$-$C_7$-Cycloalkyl; Phenyl oder Benzyl ist;
$R^{13}$ und $R^{14}$, unabhängig voneinander, $C_1$-$C_{18}$-Alkyl; $C_4$-$C_{36}$-Alkyl, welches durch $-CO-N(R^{17})-$ oder $-N(R^{17})-CO-$ unterbrochen ist; $C_5$-$C_8$-Cycloalkyl, welches unsubstituiert oder durch $-R^{12}$ substituiert ist; Phenyl, welches unsubstituiert oder durch 1 bis 4 $-R^{12}$ substituiert ist; oder $C_7$-$C_{12}$-Aralkyl bedeutet;
$R^{15}$ und $R^{16}$, unabhängig voneinander, H; oder $C_1$-$C_{12}$-Alkyl; oder gemeinsam geradkettiges, $\alpha,\omega$-verknüpftes $C_4$-$C_{13}$-Alkylen bedeuten;
$R^{17}$ Wasserstoff ist oder eine der Bedeutungen von $R^{11}$ hat;
X im Fall m = 0 beziehungsweise n = 0 Phenylen oder in 5-Stellung durch $-Z^4$ oder $-Z^5$ substituiertes 1,3-Phenylen ist;
X im Fall m = 1 beziehungsweise n = 1 eine direkte Bindung; $C_1$-$C_4$-Alkylen; Phenylen; oder $-Z^2-Z^3-$ bedeutet,

wobei $Z^2$ an das Kohlenstoffatom der Polymerkette bindet; und $Z^2$ bis $Z^5$ die oben angegebenen Bedeutungen haben.

Besonders bevorzugt ist ein Homo- oder Copolymer enthaltend 50 bis 100 Mol-% Struktureinheiten der Formel I und/oder II, worin

A Methylen und E eine Gruppe der Formel

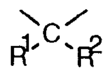

ist;

$R^2$-$N(R^{13})R^{14}$; oder $C_2$-$C_{18}$-Alkoxy; $C_4$-$C_{36}$-Alkoxy, welches durch -CO-N($R^{17}$)- oder -N($R^{17}$)-CO- unterbrochen ist; oder $C_7$-$C_{12}$-Aralkoxy bedeutet; oder

$R^1$ und $R^2$ gemeinsam einen Substituenten =O bedeuten;

$R^3$ die Bedeutung $C_1$-$C_{12}$-Alkyl; $C_4$-$C_{18}$-Alkoxy; $C_5$-$C_8$-Cycloalkyl; $C_5$-$C_8$-Cycloalkoxy; $C_7$-$C_{12}$-Phenylalkyl; $C_7$-$C_{12}$-Phenylalkoxy; oder -CO-$R^{11}$ hat;

zwei der Reste $R^4$, $R^5$ und $R^6$ Wasserstoff bedeuten und einer die Bedeutung Wasserstoff; $C_1$-$C_4$-Alkyl; Phenyl; oder $C_7$-$C_9$-Phenylalkyl hat;

$R^{11}$ $C_1$-$C_{18}$-Alkyl; Cyclohexyl; Phenyl; oder $C_7$-$C_9$-Phenylalkyl;

$R^{13}$ und $R^{14}$, unabhängig voneinander, $C_2$-$C_{18}$-Alkyl; $C_4$-$C_{36}$-Alkyl, welches durch -CO-N($R^{17}$)- oder -N($R^{17}$)-CO-unterbrochen ist; oder $C_7$-$C_{12}$-Aralkyl bedeuten;

$R^{17}$ Wasserstoff ist oder eine der Bedeutungen von $R^{11}$ hat;

X im Fall m = 0 beziehungsweise n = 0 Phenylen; und

X im Fall m = 1 beziehungsweise n = 1 eine direkte Bindung; $C_1$-$C_4$-Alkylen oder Phenylen darstellt.

Hervorzuheben sind vor allem solche Homo- oder Copolymere, welche 50 bis 100 Mol-% Struktureinheiten der Formel I und/oder II enthalten, und worin

A Methylen und E eine Gruppe der Formel

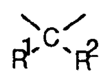

ist;

$R^2$ $C_4$-$C_{18}$-Alkoxy; $C_4$-$C_{36}$-Alkoxy, welches durch -CO-N($R^{17}$)- oder -N($R^{17}$)-CO- unterbrochen ist; oder $C_7$-$C_{12}$-Aralkoxy bedeutet; oder -N($R^{13}$)$R^{14}$ ist; oder

$R^1$ und $R^2$ gemeinsam einen Substituenten =O bedeuten;

$R^3$ die Bedeutung $C_1$-$C_6$-Alkyl; $C_4$-$C_{12}$-Alkoxy; Cyclohexyl; Cyclohexyloxy; Benzyl; Benzyloxy; oder Benzoyl hat;

zwei der Reste $R^4$, $R^5$ und $R^6$ Wasserstoff bedeuten und einer die Bedeutung Wasserstoff; Methyl; oder Phenyl hat;

$R^{13}$ und $R^{14}$, unabhängig voneinander, $C_2$-$C_{12}$-Alkyl darstellen;

X im Fall m = 0 beziehungsweise n = 0 Phenylen; und

X im Fall m = 1 beziehungsweise n = 1 eine direkte Bindung; oder Phenylen darstellt; insbesondere entsprechende Homopolymere.

Die Herstellung der genannten Homo- oder Copolymeren erfolgt zweckmäßig dadurch, daß man eine Verbindung der Formel V

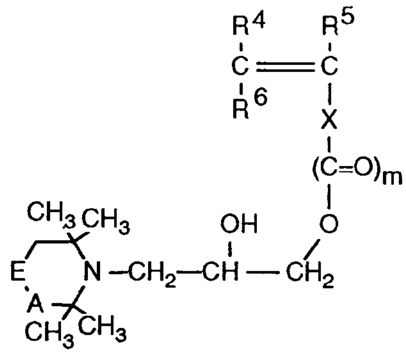

(V)

oder eine Verbindung der Formel VI

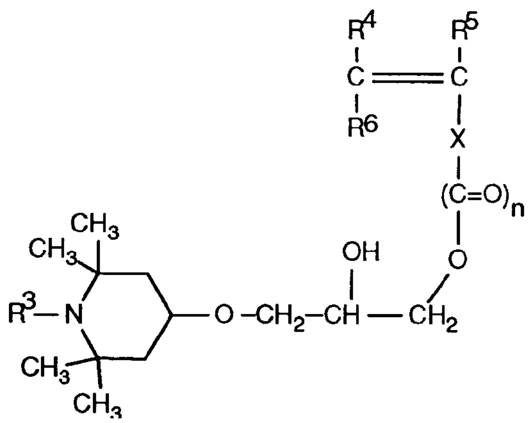

(VI)

worin A, E, $R^1$, $R^2$, $R^3$, $R^4$, $R^5$, $R^6$, m, n und X die oben angegebenen Bedeutungen haben, oder ein Gemisch aus Verbindungen der Formeln V und VI;
oder eine oder mehrere Verbindungen der Formeln V und VI zusammen mit bis zu 50 Mol-% einer anderen radikalisch polymerisierbaren Verbindung, insbesondere einer Verbindung der Formel VIII

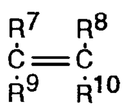

(VIII)

worin $R^7$, $R^8$, $R^9$ und $R^{10}$ die oben angegebenen Bedeutungen haben, in an sich bekannter Weise radikalisch polymerisiert. So erhältliche Polymere enthalten strukturelle Einheiten der Formel I und/oder II wie oben beschrieben und sind Gegenstand der Erfindung.

Die radikalische Polymerisation kann unter Anwendung verschiedener Techniken durchgeführt werden. Diese sind beispielsweise von S. Sandler und W. Karo beschrieben in Polymer Synthesis, Vol. 1-3, Academic Press, New York 1968. Übliche Polymerisationsverfahren sind beispielsweise Polymerisation in der Masse oder in Lösungsmitteln, Emulsions-, Suspensions- oder Fällungspolymerisation.

Die Polymerisation wird in der Regel durch einen der üblichen Initiatoren der radikalischen Polymerisation eingeleitet. Dazu zählen thermische Initiatoren wie Azoverbindungen, beispielsweise Azoisobutyronitril (AIBN), oder Peroxide, beispielsweise Benzoylperoxid, oder Redoxinitiatorsysteme, wie eine Mischung von Eisen(III)-acetylacetonat, Benzoin und Benzoylperoxid, oder photochemische Radikalbildner, wie Benzoin oder Benzilmethylketal.

Der Initiator wird der Reaktionslösung zweckmäßig in einer Menge von 0,1-5 Mol-%, vorzugsweise 0,5-3 Mol-%, zugesetzt, bezogen auf die Menge an ethylenisch ungesättigten Monomeren.

Für die Kontrolle der Molekulargewichte können Kettenübertragungsverbindungen zugesetzt werden, beispielsweise in Mengen von 5 bis 20 Mol-%. Beispiele für solche Verbindungen sind Disulfide, Mercaptane, Halogenide, Butylmercaptan, Dibutylsulfid, Tetrachlorkohlenstoff und andere.

Die Polymerisation wird bevorzugt in Lösung durchgeführt. Die Reaktionstemperatur bewegt sich im allgemeinen im Bereich von 10 bis 200°C, vorzugsweise zwischen 30 und 150°C, besonders bevorzugt zwischen

40 und 100°C.

Gegebenenfalls anwesende Lösemittel müssen unter den Reaktionsbedingungen inert sein. Als Lösemittel kommen u.a. aromatische Kohlenwasserstoffe, Ketone und Ether in Betracht. Beispiele dafür sind Benzol, Toluol, Xylol, Ethylbenzol, Isopropylbenzol, Methylethylketon, Aceton, Cyclohexanon, Diethylether, Dibutylether, Tetrahydrofuran oder Dioxan. Besonders bevorzugt sind Toluol oder Xylol.

Die Polymerisation wird zweckmäßig unter Ausschluß von Sauerstoff durchgeführt, beispielsweise unter Argon oder unter Stickstoff.

Bei den Ausgangsverbindungen der Formel V handelt es sich um neue Verbindungen, die ebenfalls einen Gegenstand der Erfindung darstellen. Auch die Ausgangsverbindungen der Formel V stellen bereits wirksame Stabilisatoren für organische Polymere zum Schutz gegen Schädigung durch Licht, Sauerstoff und/oder Wärme dar.

Dasselbe gilt für die Ausgangsverbindungen der Formel VI.

Die Ausgangsverbindungen der Formeln V und VI stellen, im Fall daß m beziehungsweise n 1 ist, beispielsweise Ester folgender Säuren dar:
Ölsäure, Acrylsäure, Methacrylsäure, Maleinsäure, Fumarsäure, Itaconsäure, Glutaconsäure, Mesaconsäure, Dodecyenlbernsteinsäure, Zimtsäure, Vinylbenzoesäure, Allylbenzoesäure, Maleimidobenzoesäure, Stilben-4,4'-dicarbonsäure, Vinylphenylessigsäure.

Im Fall daß m beziehungsweise n 0 ist, sind die Ausgangsverbindungen der Formeln V und VI Phenolether, die sich beispielsweise von den folgenden Phenolen herleiten:
2-, 3- oder 4-Vinylphenol; Allylphenol; 4,4'-Dihydroxy-stilben; Vinylresorcin.

Da die Ausgangsverbindungen der Formeln V und VI polymerisierbare Stabilisatoren darstellen, kann die Stabilisierung eines Polymers auch durch radikalische Copolymerisation oder Pfropfcopolymerisation mit einer oder beiden Verbindungen der Formeln V und VI erreicht werden. Daher bildet einen weiteren Gegenstand der Erfindung ein gegen schädigenden Einfluß von Licht, Sauerstoff und/oder Wärme geschütztes Polymer, dadurch gekennzeichnet, daß es 0,01 bis 5 Mol-% strukturelle Einheiten der Formel I und/oder II enthält.

Ein Verfahren zur Herstellung einer Verbindung der Formel V geht von einer Piperidinverbindung der Formel Va

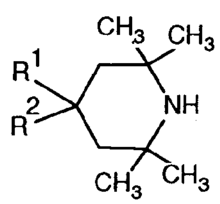

(Va)

aus; ein Verfahren zur Herstellung einer Verbindung der Formel VI geht von einer Piperidinverbindung der Formel VIa

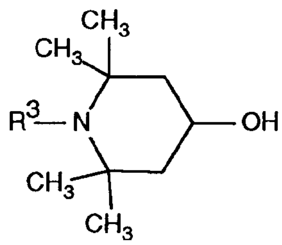

(VIa)

aus. Piperidinverbindungen dieser Art sind im allgemeinen bekannt und zum Teil kommerziell erhältlich. Ihre Herstellung ist entweder bekannt, oder sie können in Analogie zu bekannten Verfahren hergestellt werden.

Zur Herstellung einer Verbindung der Formel V oder VI kann die entsprechende Piperidinverbindung zunächst in an sich bekannter Weise unter Abspaltung von HCl mit Epichlorhydrin zum Zwischenprodukt der Formel Vb

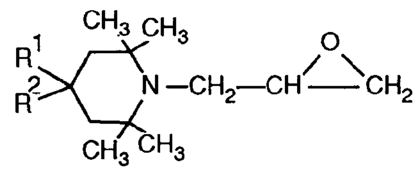

(Vb)

beziehungsweise der Formel VIb

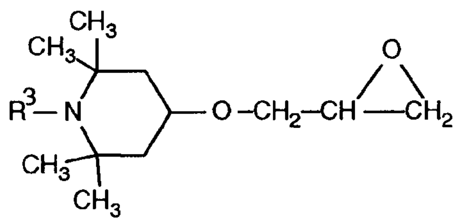

(VIb)

umgesetzt werden. Dieses kann anschließend mit einer ethylenisch ungesättigten mono- oder divalenten Carbonsäure oder einem ethylenisch ungesättigten mono- oder divalenten Phenol der Formel

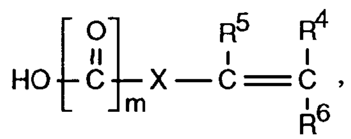

unter Bildung der Verbindung der Formel V oder VI zur Reaktion gebracht werden. Die Symbole m, $R^1$, $R^2$, $R^3$, $R^4$, $R^5$ und $R^6$ haben dabei die weiter oben angegebenen Bedeutungen.

Die Herstellung des epoxidierten Zwischenproduktes der Formel Vb oder VIb kann entsprechend oder in Analogie zu einer der Methoden erfolgen, die in der EP-A-001835 oder bei Luston und Vass, Makromol. Chem., Macromol. Symp. 27, 231 (1989), beschrieben sind. Zweckmäßig wird die Piperidinverbindung der Formel Va oder VIa in Gegenwart starker Basen, beispielsweise wässriger konzentrierter Alkalihydroxidlösung, und eines organischen Lösemittels langsam mit einem Überschuß Epichlorhydrin versetzt.

Vorteilhaft wird die Base in ca. 2-20-fachem molarem Überschuß bezogen auf die Verbindung der Formel Va oder VIa eingesetzt; beispielsweise werden 3-15 Mol, bevorzugt 4-12 Mol Natrium- oder Kaliumhydroxid als 50-% wässrige Lösung pro Mol Piperidinverbindung verwendet. Die Menge des organischen Lösemittels wird zweckmäßig so bemessen, daß die Verbindung der Formel Va oder VIa vollständig gelöst wird; als Lösemittel eignen sich beispielsweise wenig bis unpolare Lösemittel wie Kohlenwasserstoffe oder Ether; bevorzugt ist Toluol.

Auf ein Äquivalent der Piperidinverbindung der Formel Va oder VIa können beispielsweise 1-4, vorzugsweise 1,2-3, vor allem 1,5-2,5 Äquivalente Epichlorhydrin eingesetzt werden. Darüberhinaus können der Mischung vorteilhaft 1-30 Mol-%, bevorzugt 5-25 Mol-%, eines tert. Aminsalzes, beispielsweise eines Tetraalkylammoniumhalogenids wie Tetramethylammoniumchlorid oder Tetrabutylammoniumbromid, oder eines Phosphoniumsalzes, beispielsweise eines quaternären Phosphoniumhalogenids wie Ethyltriphenylphosphoniumbromid, als Katalysator zugesetzt werden.

Die Temperatur beträgt während der Reaktion zweckmäßig 0-100°C, vorzugsweise 20-80°C, vor allem 30-70°C.

Nach vollständiger Reaktion kann die Aufarbeitung nach gängigen Methoden erfolgen; zweckmäßig wird die Mischung zunächst mit Wasser verdünnt, beispielsweise indem das Reaktionsgemisch in das 1-4-fache Volumen Eiswasser gegeben wird; anschließend kann die organische Phase direkt abgetrennt oder extrahiert werden, zur Extraktion eignet sich z.B. Essigester. Das Produkt kann nach Trocknen der organischen Phase durch Entfernen des Lösemittels isoliert werden. Möglich sind auch das Einschalten weiterer Reinigungsschritte wie Dispergieren von Aktivkohle, Filtrieren oder Destillieren.

Die weitere Umsetzung des Zwischenproduktes der Formel Vb oder VIb unter Öffnen des Epoxides zur Verbindung der Formel V oder VI kann mit oder ohne Lösemittel durchgeführt werden; gegebenenfalls einsetzbare Lösemittel sind polar oder unpolar, inert und bevorzugt hochsiedend. So können beispielsweise aromatische oder aliphatische Kohlenwasserstoffe ebenso verwendet werden wie heterozyklische Lösemittel, Ether, Sulfone, Sulfoxide oder Amide; bevorzugte Lösemittel sind unter anderem höhersiedende Kohlenwasserstofffraktionen wie Ligroin oder Petrolether, aromatische Kohlenwasserstoffe wie Toluol oder Xylol, Dekalin, cyclische oder offenkettige Ether wie Dibutylether oder Dioxan, Dimethylformamid, Dimethylsulfoxid; besonders bevorzugt sind Toluol oder Xylol.

Die Temperatur der Reaktionsmischung kann für die Dauer der Reaktion im Siedebereich gehalten werden (Rückfluß). Dazu wird eine Lösemittel enthaltende Reaktionsmischung, im Allgemeinen unter Normaldruck, zum Siedepunkt erwärmt und das verdampfte Lösemittel mit Hilfe eines geeigneten Kühlers kondensiert und wieder der Reaktionsmischung zugeführt. Der Siedebereich des reinen Lösemittels kann gegebenenfalls im Bereich von 60-180°C liegen, beispielsweise 60-140°C. Wird zur Umsetzung eine Carbonsäure eingesetzt, wird die Temperatur bevorzugt im Bereich 30-130°C, vor allem 40-90°C gehalten, die Reaktionsdauer beträgt beispielsweise 1 bis 20 Stunden. Im Falle des Einsatzes eines Phenols wird die Temperatur bevorzugt im Bereich 80-180°C, vor allem 100-160°C gehalten, die Reaktionsdauer beträgt beispielsweise 3 bis 36 Stunden.

Die Reaktion wird vorzugsweise unter Schutzgas, z.B. Stickstoff oder Argon, durchgeführt; die Reaktionsmischung wird zweckmäßig gerührt. Das Epoxid der Formel Vb oder VIb wird vorzugsweise in etwa äquivalenter Menge oder in leichtem Überschuß bezogen auf die Carbonsäuregruppen oder die phenolischen Hydroxylgruppen des Reaktanden eingesetzt, beispielsweise in einer Menge von 1,0 bis 1,3 Äquivalente, vor allem 1,0 bis 1,15 Äquivalente pro Äquivalent phenolisches OH oder Carbonsäure.

Die Reaktion wird vorzugsweise in Anwesenheit eines tert. Aminsalzes, beispielsweise eines Tetraalkylammoniumhalogenids wie z.B. Tetramethylammoniumchlorid oder Tetrabutylammoniumbromid, oder eines Phosphoniumsalzes, beispielsweise eines quaternären Phosphoniumhalogenids wie Ethyltriphenylphosphoniumbromid, als Katalysator durchgeführt. Der Katalysator wird zweckmäßig in einer Menge von 1 bis 5 Mol-% bezogen auf die Verbindung der Formel Vb oder VIb eingesetzt.

Die Aufarbeitung nach Beendigung der Reaktion kann nach gängigen Methoden erfolgen; beispielsweise wird die erkaltete Mischung zunächst mit der wässrigen Lösung einer Base, z.B. stark verdünnter Alkalilauge, und anschließend mit Wasser gewaschen und nach Trocknen das Lösemittel entfernt, z.B. durch Anlegen von Unterdruck und/oder Erwärmen. Nach dem Trocknen können noch weitere Reinigungsschritte wie Dispergieren von Aktivkohle, Filtrieren, Destillieren usw. eingeschaltet werden.

In einem weiteren möglichen Herstellungsverfahren für die erfindungsgemäßen Verbindungen der Formel V oder VI wird Epichlorhydrin zunächst mit der Carbonsäure beziehungsweise mit dem Phenol umgesetzt, und das erhaltene Zwischenprodukt anschließend unter Öffnen des Epoxidringes mit der Piperidinverbindung der Formel Va oder VIa zur gewünschten Verbindung der Formel V oder VI reagiert.

Verbindungen der Formel Vb lassen sich weiterhin durch Oxidation der entsprechenden N-Allylverbindungen mit Hilfe von Persäuren, beispielsweise Peressigsäure, erhalten. Die weitere Umsetzung kann erfolgen wie oben beschrieben.

Homo- oder Copolymere enthaltend strukturelle Einheiten der Formel I und/oder II sowie Verbindungen der Formel V und der Formel VI eignen sich zum Stabilisieren von organischen Polymeren gegen thermischen, oxidativen und aktinischen Abbau.

Beispiele für derartige Materialien sind:

1. Polymere von Mono- und Diolefinen, beispielsweise Polypropylen, Polyisobutylen, Polybuten-l, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).

Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:

a) radikalisch (gewöhnlich bei hohem Druck und hoher Temperatur).

b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder $\pi$- oder $\sigma$-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme

werden gewöhnlich als Phillips, Standard Oil Indiana, Ziegler (-Natta), TNZ (DuPont), Metallocen oder Single Site Katalysatoren (SSC) bezeichnet.

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten- 1 -Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.

4. Kohlenwasserstoffharze (z.B. $C_5$-$C_9$) inklusive hydrierte Modifikationen davon (z.B. Klebrigmacherharze) und Mischungen von Polyalkylenen und Stärke.

5. Polystyrol, Poly-(p-methylstyrol), Poly-($\alpha$-methylstyrol).

6. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

7. Pfropfcopolymere von Styrol oder $\alpha$-Methylstyrol, wie z.B. Styrol auf Polybutadien, Styrol auf Polybutadien- Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.

8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

9. Polymere, die sich von $\alpha,\beta$-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-VinylhalogenidCopolymere oder Acrylnitril-Alkylmethacrylat-Butadien-Terpolymere.

11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

12. Homo- und Copolymere von cyclischen Ethern, wie Polyalkylenglykole, Polyethylenoxyd, Polypropylenoxyd oder deren Copolymere mit Bisglycidylethern.

13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

14. Polyphenylenoxide und -sulfide und deren Mischungen mit Styrolpolymeren oder Polyamiden.

15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

17. Polyharnstoffe, Polyimide, Polyamid-imide und Polybenzimidazole.

18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

19. Polycarbonate und Polyestercarbonate.

20. Polysulfone, Polyethersulfone und Polyetherketone.

21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

22. Trocknende und nicht-trocknende Alkydharze.

23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

24. Vernetzbare Acrylharze, die sich von substituierten Acrylsäureestern ableiten, wie z.B. von Epoxyacrylaten, Urethan-acrylaten oder Polyester-acrylaten.

25. Alkydharze, Polyesterharze und Acrylatharze, die mit Melaminharzen, Harnstoffharzen, Polyisocyanaten oder Epoxidharzen vernetzt sind.

26. Vernetzte Epoxidharze, die sich von Polyepoxiden ableiten, z.B. von Bis-glycidylethern oder von cycloaliphatischen Diepoxiden.

27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Weitere Gegenstände der Erfindung sind daher Zusammensetzungen enthaltend (a) ein gegen Schädigung durch Licht, Sauerstoff und/oder Wärme empfindliches organisches Polymer und (b) als Stabilisator ein Polymer enthaltend strukturelle Einheiten der Formel I und/oder II, sowie die Verwendung der oben genannten Polymeren oder Copolymeren zum Stabilisieren von organischen Polymeren gegen Schädigung durch Licht, Sauerstoff und/oder Wärme.

Die Erfindung betrifft ebenfalls ein Verfahren zum Stabilisieren organischer Polymere gegen Schädigung durch Licht, Sauerstoff und/oder Wärme, dadurch gekennzeichnet, daß man den Polymeren als Stabilisator ein Polymer oder Copolymer enthaltend strukturelle Einheiten der Formel I und/oder II oder eine Verbindung der Formel V oder der Formel VI, oder ein Gemisch solcher monomerer oder polymerer Verbindungen beimischt. Ein bevorzugter Gegenstand der Erfindung ist ein Verfahren zum Stabilisieren organischer Polymere gegen Schädigung durch Licht, Sauerstoff und/oder Wärme, dadurch gekennzeichnet, daß man den Polymeren als Stabilisator ein Polymer oder Copolymer enthaltend strukturelle Einheiten der Formel I und/oder II beimischt.

Von besonderem Interesse ist die Verwendung der erfindungsgemäßen Polymere oder Copolymere als Stabilisatoren in synthetischen organischen Polymeren, insbesondere Thermoplasten, beispielsweise Polyolefinen.

Vorzugsweise handelt es sich bei den zu schützenden organischen Polymeren um natürliche, halbsynthetische oder bevorzugt synthetische organische Polymere. Besonders bevorzugt sind synthetische organische Polymere oder Gemische solcher Polymere, insbesondere thermoplastische Polymere wie Polyolefine, vor allem Polyethylen und Polypropylen (PP). Ebenfalls besonders bevorzugte organische Materialien sind

Überzugszusammensetzungen. Unter photographischen Materialien sind insbesondere die Materialien zu verstehen, die in Research Disclosure 1990, 31429 (Seiten 474-480) für die photographische Reproduktion und andere Reproduktionstechniken beschrieben sind. Im Sinne der Erfindung vorteilhaft zu stabilisierende Überzugszusammensetzungen sind beispielsweise beschrieben in Ullmann's Encyclopedia of Industrial Chemistry, 5. Ed., Vol. A18, pp. 359-464, VCH Verlagsgesellschaft, Weinheim 1991.
Zusammensetzungen, worin die zu schützende Komponente (a) ein Polyolefin oder ein Lackbindemittel auf der Basis von Acryl-, Alkyd-, Polyurethan-, Polyester- oder Polyamidharz oder entsprechender modifizierter Harze ist, stellen daher einen besonders bevorzugten Gegenstand der Erfindung dar.

Allgemein werden die erfindungsgemäßen Polymeren oder Copolymeren, oder die erfindungsgemäßen Monomeren der Formel V oder VI, dem zu stabilisierenden Material in Mengen von 0,01 bis 10 %, bevorzugt 0,01 bis 5 %, insbesondere 0,01 bis 2 %, bezogen auf das Gesamtgewicht der stabilisierten Zusammensetzung, zugesetzt. Besonders bevorzugt ist der Einsatz der erfindungsgemäßen Verbindungen in Mengen von 0,05 bis 1,5 %, vor allem 0,1 bis 1,5 %.

Die Einarbeitung in die Materialien kann beispielsweise durch Einmischen oder Aufbringen der erfindungsgemäßen Polymere oder Copolymere und gegebenenfalls weiterer Additive nach den in der Technik üblichen Methoden erfolgen. Beispielsweise kann die Einarbeitung in die zu schützenden Polymere vor oder während der Formgebung, oder durch Aufbringen der gelösten oder dispergierten Verbindung auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der erfindungsgemäßen Polymere oder Copolymere besteht in deren Zugabe vor, während oder unmittelbar nach der Polymerisation der entsprechenden Monomeren bzw. vor der Vernetzung. Die erfindungsgemäßen Polymere oder Copolymere können dabei als solche, aber auch in encapsulierter Form (z.B in Wachsen, Ölen oder weiteren Polymeren) zugesetzt werden. Im Falle der Zugabe vor oder während der Polymerisation können die erfindungsgemäßen Polymere oder Copolymere auch als Regulator für die Kettenlänge der Polymere (Kettenabbrecher) wirken.

Die erfindungsgemäßen Polymere oder Copolymere können auch in Form eines Masterbatches, der diese Verbindung beispielsweise in einer Konzentration von 2,5 bis 25 Gew.-% enthält, den zu stabilisierenden Kunststoffen zugesetzt werden.

Eine besondere Form solcher Masterbatches stellen Pfropfcopolymere dar, welche 2 bis 20 Mol-% strukturelle Einheiten der Formel I und/oder II enthalten. Dabei finden besonders solche Pfropfcopolymere Verwendung, deren Rückgrat aus einem Polyolefin, beispielsweise aus Polyethylen oder Polypropylen, und dessen Seitenketten aus strukturellen Einheiten der Formel I und/oder II gebildet sind.

Zweckmäßig kann die Einarbeitung der erfindungsgemäßen Polymere oder Copolymere nach folgenden Methoden erfolgen:

- als Emulsion oder Dispersion (z.B. zu Latices oder Emulsionspolymeren),
- als Trockenmischung während des Vermischens von Zusatzkomponenten oder Polymermischungen,
- durch direktes Zugeben in die Verarbeitungsapparatur (z.B. Extruder, Innenmischer usw.),
- als Lösung oder Schmelze.

Erfindungsgemäße Polymerzusammensetzungen können in verschiedener Form angewendet bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Neben den erfindungsgemäßen Polymeren oder Copolymeren können die erfindungsgemäßen Zusammensetzungen zusätzlich herkömmliche Additive enthalten, wie beispielsweise die unten angegebenen.

Die herkömmlichen Additive werden zweckmäßig in Mengen von 0,1-10, beispielsweise 0,2-5 Gew.-%, bezogen auf das zu stabilisierende Polymer, eingesetzt.

1. Antioxidantien

1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.

1.4. Tocopherole, z.B. α-Tocopherol, β-Tocopherol, γ-Tocopherol, δ-Tocopherol und Mischungen davon (Vitamin E).

1.5. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.

1.6. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, Isooctyl-3,5-di-tert-butyl-4-hydroxybenzyl-mercaptoacetat.

1.8. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.

1.9. Hydroxybenzyl-Aromaten, z.B.1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.

1.10. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.

1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.13. Ester der β-(3,5-Di-tert-butyl-4-hydroxyphenyl)-propionsäure mit ein- oder mehr wertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(5-tert-Butyl-4-hydroxy-3-methylphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der β-(3,5-Dicyclohexyl-4-hydroxyphenyl)-propionsäure mit ein- oder mehr wertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.17. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

2. UV-Absorber und Lichtschutzmittel

2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl- 2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4-octoxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, Mischung aus 2-(3'-tert-Butyl-2'-hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylenbis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; $[R-CH_2CH_2-COO(CH_2)_3]_2$ mit R = 3'-tert-Butyl-4'-hydroxy-5'-2H-benzotriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

8. Nukleierungsmittel, wie z.B. 4-tert-Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.

10. Sonstige Zusätze, wie z.B. Weichmacher, Gleitmittel, Emulgatoren, Pigmente, Optische Aufheller, Flammschutzmittel, Antistatika, Treibmittel.

11. Benzofuranone bzw. Indolinone, wie z.B. in US-A-4 325 863, US-A-4 338 244, US-A-5 175 312, US-A-5 216 052, US-A-5 252 643, DE-A-4 316 611, DE-A-4 316 622, DE-A-4 316 876, EP-A-0 589 839 oder EP-A-0 591 102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis-[5,7-di-tert-butyl-3-(4-[2-hydroxyethoxy]-phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on.

Die nachfolgenden Beispiele illustrieren die Erfindung weiter. Alle Angaben in Teilen oder Prozenten, in den Beispielen ebenso wie in der übrigen Beschreibung sowie in den Patentansprüchen, beziehen sich auf das Gewicht, sofern nichts anderes angegeben ist. In den Beispielen werden die folgenden Abkürzungen verwendet:

DMF: Dimethylformamid;

THF: Tetrahydrofuran;
GC: Gaschromatographie;
HPLC Hochdruck-Flüssigchromatographie;
GPC: Gelpermeationschromatographie;
DSC: Differential-Thermoanalyse;
TGA: Thermogravimetrische Analyse;
AIBN: α,α'-Azoisobutyronitril;
$M_n$: Zahlenmittel der Molmasse (Einheit g/Mol);
$M_w$: Massenmittel der Molmasse (Einheit g/Mol).

Herstellungsbeispiele

A) Herstellung der Monomeren

A1) 4-(3-Acryloxy-2-hydroxypropyloxy)-1,2,2,6,6-pentamethylpiperidin

A1a) 1,2,2,6,6-Pentamethyl-4-(2,3-epoxypropoxy)piperidin

In einem 2,5 1 Sulfierkolben mit mechanischem Rührer, Kühler und 500 ml Tropfrichter werden unter Argonatmosphäre 300 g (7,5 Mol) Natriumhydroxyd in 300 g Wasser gelöst. Dazu gibt man 750 ml Toluol, 48,4 g (0,15 Mol) Tetrabutylammoniumbromid und 257 g (1,5 Mol) 4-Hydroxy-1,2,2,6,6-pentamethylpiperidin.

Bei 60°C werden 347 g Epichlorhydrin (3,75 Mol) in 1,5 Stunden zugetropft, anschließend wird bei gleicher Temperatur für weitere 4 Std. gerührt.

Die Reaktionslösung wird auf 3 l Eiswasser gegossen, die organische Phase abgetrennt, mit Natriumsulfat getrocknet und eingedampft.

Bei 0,05 Torr wird über eine Vigreuxkolonne destilliert und die Fraktion mit Siedepunkt 71-72°C gesammelt.

Ausbeute: 205 g (60 %) GC: >99 %

| Mikroanalyse | | |
|---|---|---|
| | berechnet | gefunden |
| C | 68.68 | 68.64 |
| H | 11.07 | 11.21 |
| N | 6.16 | 6.32 |
| Cl | 0.0 | 0.0 |

$^1H$-NMR ($CDCl_3$):
1,02 und 1,16 ppm (12 H,s): $CH_3$-Gruppen des Piperidinrings
1,32-1,4 ppm und 1,83-1,91 ppm (4 H, m): $-CH_2$-Gruppen des Piperidinrings
2,23 ppm (3 H, s): $N-CH_3$
2,60-2,62 ppm und 2,78-2,82 ppm (2 H, m): $CH_2$-Gruppe des Epoxidrings
3,42-3,47 ppm und 3,71-3,76 ppm (2 H, m): $0-CH_2$-Gruppe
3,57-3,67 ppm (1 H, m): CH-O des Piperidinrings

A1b) 4-(3-Acryloxy-2-hydroxypropyloxy)-1,2,2,6,6-pentamethylpiperidin

In einem 2,5 l Sulfierkolben mit Magnetrührer, Thermometer und Kühler werden unter Argon 375 g (1,65 Mol) 4-(2,3-Epoxypropoxy)-1,2,2,6,6-pentamethylpiperidin (= A1a) in 1,5 1 Xylol vorgelegt. Dazu gibt man 108 g (1,5 Mol) Acrylsäure und 27,8 g (75 mmol) Ethyltriphenylphosphoniumbromid. Bei 70°C lässt man während 18 Stunden reagieren. Das abgekühlte Produkt giesst man auf Eiswasser und trennt die org. Phase ab. Die organische Phase wird zweimal mit 1 n Natronlauge gewaschen, getrocknet und eingedampft.

Der Rückstand wird über eine kurze Vigreuxkolonne destilliert. Man erhält 193 g (43 %) des Titelproduktes als klare Flüssigkeit vom Siedepunkt 119-125°C bei 0,06 Torr.

| Mikroanalyse | | |
|---|---|---|
| | berechnet | gefunden |
| C | 64.18 | 63.50 |
| H | 9.76 | 9.76 |
| N | 4.68 | 4.67 |

$^1$H-NMR ($CDCl_3$):
1,02 und 1,16 ppm (12 H,s): $CH_3$ (Piperidin)
1,33-1,40 und 1,84-1,89 ppm (4 H, m): $CH_2$ (Piperidin)
2,23 ppm (3 H, s): N-$CH_3$
2,59 ppm (1 H, s): OH
3,46-3,63 und 3,71-3,72 und 3,84-3,86 und 3,98-4,05 und 4,18-4,29 ppm (6 H, m):

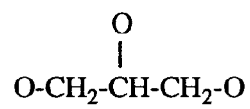

und CH (Piperidin)
5,84-5,88 und 6,12-6,22 und 6,42-6,48 ppm (3 H, m): CO-CH=$CH_2$

A2) 4-(3-Methacryloxy-2-hydroxypropyloxy)-1,2,2,6,6-pentamethylpiperidin

In einem 1,5 l Sulfierkolben mit Magnetrührer, Thermometer und Kühler werden unter Schutzgas 250 g (1,1 Mol) 4-(2,3-Epoxypropoxy)-1,2,2,6,6-pentamethylpiperidin in 1 l Xylol gelöst. Dazu gibt man 86 g (1 Mol) Methacrylsäure und 18,6 g (50 mmol) Ethyltriphenylphosphoniumbromid.

Die Mischung wird bei 70°C während 18 Stunden gerührt. Nach dem Abkühlen giesst man auf Eiswasser und trennt die organische Phase ab. Diese wird zweimal mit 1 n Natronlauge gewaschen und getrocknet. Das Lösungsmittel wird dem Rotavap abgedampft und der Rückstand bei 0,07 Torr destilliert. Man erhält 176,5 g (56 %) einer klaren Flüssigkeit, welche bei 128-130°C siedet.

| Mikroanalyse | | |
|---|---|---|
| | berechnet | gefunden |
| C | 64.14 | 64.72 |
| H | 9.97 | 10.00 |
| N | 4.47 | 4.33 |

$^1$H-NMR ($CDCl_3$):
1,01 und 1,16 ppm (12 H,s): $CH_3$ (Piperidin)
1,32-1,40 und 1,83-1,88 ppm (4 H, m): $CH_2$ (Piperidin)
1,96 ppm (3 H, s): $CH_3$ (Methacrylat)
2,23 ppm (3 H, s): $CH_3$-N
2,68 ppm (1 H, s): OH
3,46-4,26 ppm (6 H, m):

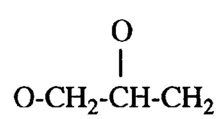

und CH (Piperidin)
5,59 und 6,14 ppm (2 H, s): $CH_2$=C

A3) 4-(3-[4-Vinylbenzoyloxy]-2-hydroxypropyloxy)-1,2,2,6,6-pentamethylpiperidin

In einem 500 ml Rundkolben, ausgerüstet mit Magnetrührer, Kühler, Thermometer und Argonballon werden 32,6 g (220 mmol) 4-Vinylbenzoesäure, 45,5 g (200 mmol) 4-(2,3-Epoxypropoxy)-1,2,2,6,6-pentamethylpiperidin, 3,7 g (10 mmol) Ethyltriphenylphosphoniumbromid und 200 ml Xylol vorgelegt. Unter Schutzgas wird die Mischung auf 65°C erwärmt. Die klare Lösung wird während 15 Stunden bei 65°C gehalten und dann auf Eis gegossen. Die organische Phase wird bei 0°C zweimal mit 1 n Natronlauge gewaschen, über Natriumsulfat getrocknet und eingedampft.
Der Rückstand wird über eine Kieselgelsäule (THF) gereinigt und am Hochvakuum bei 60°C getrocknet. Man erhält 52,7 g (70 %) einer klaren, viskosen Flüssigkeit.

| Mikroanalyse | | |
|---|---|---|
| | berechnet | gefunden |
| C | 70.37 | 69.89 |
| H | 8.86 | 9.22 |
| N | 3.73 | 3.79 |

$^1$H-NMR ($CDCl_3$):
1,01 und 1,15 ppm (12 H,s): $CH_3$
1,33-1,43 und 1,84-1,89 ppm (4 H, m): $CH_2$ (Piperidin)
2,23 ppm (3 H, s): N-$CH_3$
2,81 ppm (1 H, s): OH
3,42-4,40 ppm (6 H, m):

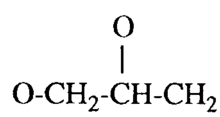

und CH (Piperidin)
5,36-5,40 ppm und 5,83-5,89 ppm (2 H, d): $CH_2$=
6,70-6,79 ppm (1 H, q): CH=
7,44-7,47 ppm und 7,99-8,02 ppm (4 H, d): Aromat

A4) 1-(3-[4-Vinylbenzoyloxy]-2-hydroxypropyl)-4-benzyloxy-2,2,6,6-tetra-methylpiperidin

In einem 500 ml Rundkolben mit Magnetrührer, Thermometer, Kühler und Argonballon werden 53 g (175 mmol) 1-(2,3-Epoxypropyl)-4-benzyloxy-2,2,6,6-tetramethyl piperidin, 28,5 g (192 mmol) 4-Vinylbenzoesäure, 3,2 g (8,7 mmol) Ethyltriphenylphosphoniumbromid und 200 ml Xylol vorgelegt.
Man lässt während 15 Stunden bei 65°C rühren und giesst dann auf Eis. Nach dem Aufarbeiten gemäss der vorherigen Beispiele erhält man nach der Säulenchromatographie (Kieselgel; Hexan/Essigester = 3:1) eine farblose, viskose Flüssigkeit.
Ausbeute: 55,1 g (70 %)

| Mikroanalyse | | |
|---|---|---|
| | berechnet | gefunden |
| C | 74.47 | 74.11 |
| H | 8.26 | 8.26 |
| N | 3.10 | 3.08 |

$^1$H-NMR ($CDCl_3$):
0,99-1,21 ppm (12 H,s): $CH_3$
1,50 ppm und 1,96 ppm (4 H, m): $CH_2$ (Piperidin)
2,52-2,60 ppm und 2,72-2,86 ppm (2 H, m): N-$CH_3$
3,72-3,81 ppm (2 H, m): =CH-OH

4,17-4,23 ppm (1 H, m): CH (Piperidin)
4,35-4,40 ppm (2 H, s): $CH_2$-O-Aromat
5,35-5,39 ppm und 5,83-5,88 ppm (2 H, d): $CH_2$=
6,69-6,79 ppm (1 H, q): CH=
7,25-7,35 ppm (5 H, m): Benzylaromat
7,43-7,46 und 8,01-8,04 ppm (4 H, d): Benzoesäurearomat

A5) 1(3-Acryloxy-2-hydroxypropyl)-4-benzyloxy-2,2,6,6-tetramethylpiperidin

Analog A4) werden 50 g (165 mMol) 1-(2,3-Epoxypropyl)-4-benzyloxy-2,2,6,6-tetramethylpiperidin, 13,1 g (181 mMol) Acrylsäure, 3,06 g (8,3 mMol) Ethyltriphenylphosphoniumbromid in 200 ml Xylol umgesetzt. Das Reaktionsprodukt wird mit Natriumhydroxidlösung bei 0°C gewaschen und aufgearbeitet. Man erhält 39 g (63 %) klare, viskose Flüssigkeit.

| Mikroanalyse: | | | |
|---|---|---|---|
| | % C | % H | % N |
| berechnet | 70,37 | 8,86 | 3,73 |
| gefunden | 70,40 | 8,97 | 3,49 |

$^1$H-NMR ($CDCl_3$):
1,00-1,29 ppm (12H, m): $CH_3$
1,49 und 1,97 ppm (4H, m): $CH_2$ (Piperidin)
2,46-2,54 ppm und 2,67-276 ppm (2H, m): N-$CH_2$
3,70-3,79 ppm (2H, m): >CH-OH
4,00-4,06 ppm (1H, m): CH (Piperidin)
4,22-4,46 ppm (2H, m): COO-$CH_2$-
4,56 ppm (2H, s): O-$CH_2$-Aromat
5,82-5,85 ppm und 6,12-6,21 ppm und 6,42-6,48 ppm (3H, m): Acryl-Gruppe
7,26-7,35 ppm (5H, m): Aromat

A6) 1(3-Methacryloxy-2-hydroxypropyl)-4-benzyloxy-2,2,6,6-tetramethylpiperidin

Die Herstellung erfolgt analog A5); man erhält 54 g (84 %) einer klaren, viskosen Flüssigkeit.

| Mikroanalyse: | | | |
|---|---|---|---|
| | % C | % H | % N |
| berechnet | 70,92 | 9,06 | 3,60 |
| gefunden | 70,89 | 9,08 | 3,47 |

$^1$H-NMR ($CDCl_3$):
1,00-1,21 ppm (12H, m): $CH_3$ (Piperidin)
1,48 ppm (2H, m): $CH_2$ (Piperidin)
1,94-1,96 ppm (5H, m): $CH_2$ (Piperidin), $CH_3$ (Methacrylat)
2,47-2,55 und 2,67-2,74 ppm (2H, m): N-$CH_2$-
3,71-3,79 ppm (2H, m): >CH-OH
4,00-4,06 ppm (1H, m): CH (Piperidin)
4,18-4,28 ppm (2H, m): -COO-$CH_2$-
4,55 ppm (2H, s): O-$CH_2$-Aromat
5,58 ppm und 6,15 ppm (2H, s): $CH_2$=C
7,26-7,34 ppm (5H, m): Aromat

A7) 1(3-[4-Vinylphenoxy]-2-hydroxypropyl)-4-benzyloxy-2,2,6,6-tetramethylpiperidin

In einem 500 ml Sulfierkolben mit Magnetrührer, Kühler und Argon-Ballon werden 43,3 g (360 mMol) 4-Hydroxystyrol, 91 g (300 mMol) 1-(2,3-Epoxypropyl)-4-benzyloxy-2,2,6,6-tetramethylpiperidin, 5,6 g (15 mMol) Ethyltriphenylphosphoniumbromid, 1,3 g (6 mMol) Di-tert.butylkresol und 250 ml Dioxan vorgelegt. Unter Rühren wird die Reaktionsmischung für 14 Stunden auf Rückflußtemperatur gehalten, anschließend auf Eis/ Essigester gegeben, die organische Phase zweimal mit 1n NaOH bei 0°C gewaschen, getrocknet und eingeengt. Der Festkörper wird in n-Hexan umkristallisiert.

Man erhält 100 g (78 %) einer farblosen Substanz, die bei 86°C schmilzt.

| Mikroanalyse: | | | |
|---|---|---|---|
| | % C | % H | % N |
| berechnet | 76,56 | 8,80 | 3,31 |
| gefunden | 76,49 | 8.84 | 3,19 |

$^1$H-NMR ($ODCl_3$):
0,96-1,18 ppm (12H, m): $CH_3$
1,38-1,50 und 1,95-2,02 ppm (4H, m): $CH_2$ (Piperidin)
2,62-2,85 ppm (2H, m): N-$CH_2$-
3,73-4,02 ppm (3H, m): O-$CH_2$-CH
4,35 ppm (1H, s): OH
4,57 ppm (2H, s): O-$CH_2$Ar
5,10-5,14 ppm und 5,57-5,63 ppm (2H, d): $CH_2$=
6,61-6,70 ppm (1H, q): CH=
6,87-6,90 ppm und 7,25-7,35 ppm (9H, d, m): H-Aromaten

A8) 4(3-[4-Vinylphenoxy]-2-hydroxypropyloxy)-1,2,2,6,6-pentamethylpiperidin

Analog Beispiel A7) werden 28,8 g (240 mMol) 4-Vinylphenol mit 45,5 g (200 mMol) 4-(2,3-Epoxypropoxy)-1,2,2,6,6-pentamethylpiperidin umgesetzt.
Ausbeute: 66 g (95 %)

| Mikroanalyse: | | | |
|---|---|---|---|
| | % C | % H | % N |
| berechnet | 72,58 | 9,57 | 4,03 |
| gefunden | 72,67 | 9,53 | 3,89 |

$^1$H-NMR ($CDCl_3$) :
1,01 und 1,15 ppm (12H, s): $CH_3$ (Piperidin)
1,32-1,41 ppm und 1,84-1,89 ppm (4H, m): $CH_2$ (Piperidin)
2,23 ppm (3H, s): N-$CH_3$
2,72 ppm (1H, s): OH
3,54-3,69 ppm (3H, m): Pip-O-$CH_2$-CH-
3,98-4,03 ppm (2H, d): ar-O-$CH_2$-
4,06-4,12 ppm (1H, m): >CH-O-
5,11-5,14 ppm und 5,58-5,64 ppm (2H, d): $CH_2$=
6,60-6,70 ppm (1H, q): CH=
6,86-6,89 ppm und 7,32-7,35 ppm (4H, d): H-Aromat

Beispiele A9-A14: Entsprechend der unter A1 beschriebenen Methode werden die Monomeren gemäß folgender Tabelle durch Umsetzung des entsprechenden Epoxids mit der entsprechenden Säure hergestellt:

| Beispiel | Monomer | Epoxid | Säure |
|---|---|---|---|
| A9 | 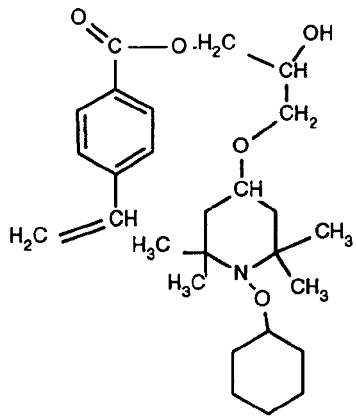 | 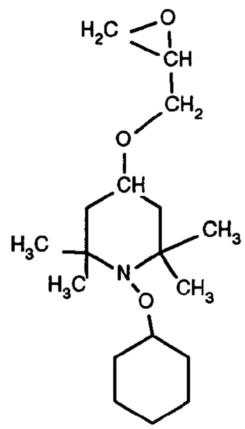 | 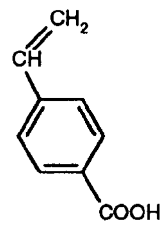 |

| Beispiel | Monomer | Epoxid | Säure |
|---|---|---|---|
| A10 | 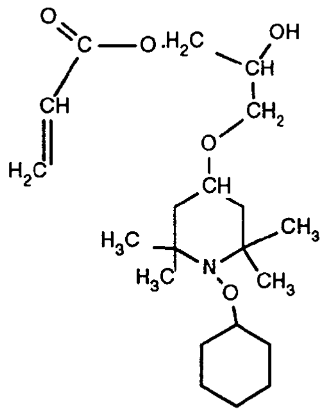 | 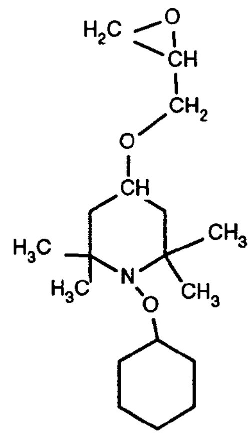 | 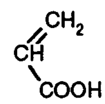 |
| A11 | 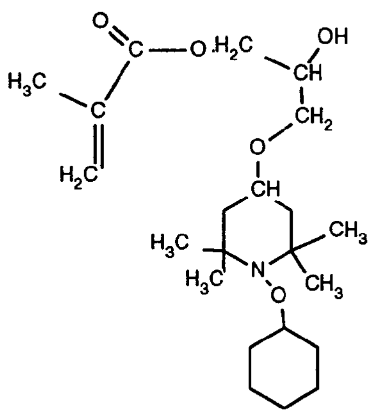 | 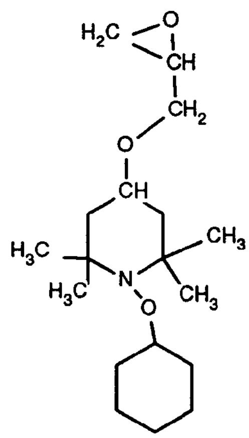 | 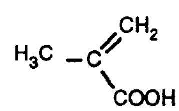 |
| A12 | 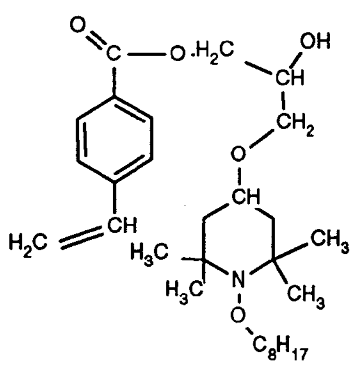 | 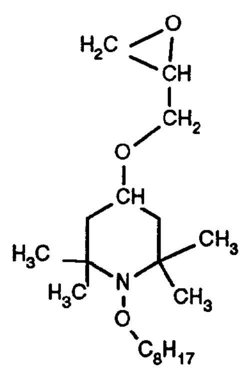 | 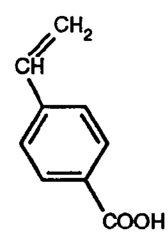 |

| Beispiel | Monomer | Epoxid | Säure |
|---|---|---|---|
| A13 | 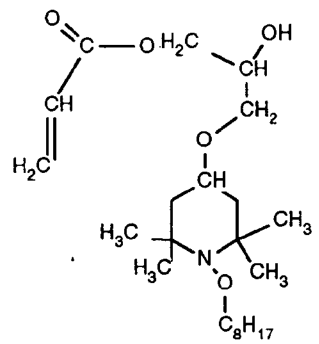 | 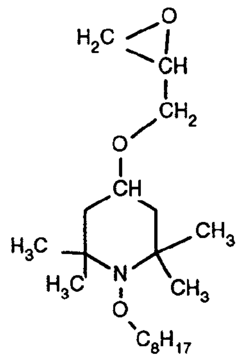 | 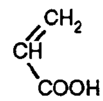 |
| A14 | 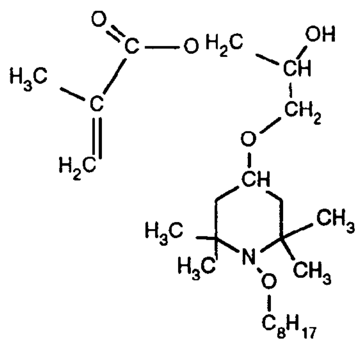 | 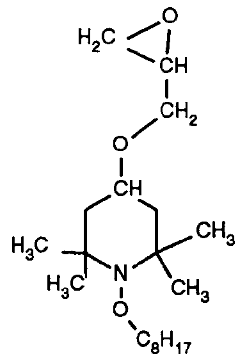 | 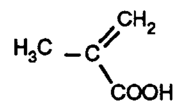 |

A15) 4(3-[4-Vinylphenoxy]-2-hydroxypropyloxy)-1-cyclohexyloxy-2,2,6,6-tetramethylpiperidin

Analog Beispiel A8) wird 4-Vinylphenol mit 4-(2,3-Epoxypropoxy)-1-cyclohexyloxy-2,2,6,6-tetramethylpiperidin unter Bildung des Titelproduktes umgesetzt.

A16) 4(3-[4-Vinylphenoxy]-2-hydroxypropyloxy)-1-octyloxy-2,2,6,6-tetramethylpiperidin

Analog Beispiel A8) wird 4-Vinylphenol mit 4-(2,3-Epoxypropoxy)-1-octyloxy-2,2,6,6-tetramethylpiperidin unter Bildung des Titelproduktes umgesetzt.

B) Herstellung der Polymeren

B1) Poly[4-(3-Acryloxy]-2-hydroxypropyl)-1,2,2,6,6-pentamethylpiperidin]

In einem 500 ml Rundkolben, versehen mit einem Magnetrührer und einem Argonballon werden 60 g (0,2 Mol) des Monomers A1), 657 mg (4 mmol) AIBN und 300 ml Toluol eingefüllt. Mittels Vakuum/Argon wird die Mischung vom Sauerstoff befreit.

Unter Argon wird bei 70°C während 15 Stunden polymerisiert. Die viskose Lösung wird auf n-Hexan gegeben und halbfestes Material fällt aus. Es wird erneut in Toluol gelöst und auf n-Hexan gefällt. Nach dem Trocknen am Hochvakuum erhält man 35,7 g (60 %) Polymer.

| Mikroanalyse | | |
|---|---|---|
| | berechnet | gefunden |
| C | 64.18 | 64.17 |
| H | 9.76 | 9.88 |
| N | 4.68 | 4.24 |

TGA ($N_2$, 10°C/Min): 5 % Masseverlust bei 320°

$^1$H-NMR ($CDCl_3$): Zeigt keine Acrylat-Doppelbindungen

GPC (DMF, 85°C): $M_n$: 50000
$M_w$: 165000

B1a) Eine weitere Polymerisation wird durchgeführt wie unter B1 beschrieben, wobei jedoch 0,37 Mol des Monomers AI, 3,7 mmol AIBN und zusätzlich als Kettenübertragungsreagens 74 mmol Butylmercaptan eingesetzt werden. Man erhält das Polymer wie unter B1, jedoch vom Molekulargewicht (GPC; DMF, 85°C) $M_n$ = 6800 und $M_w$ = 8100.

B1b) Eine weitere Polymerisation wird durchgeführt wie unter B1 beschrieben, wobei jedoch auf 60 g (0,2 Mol) des Monomers AI 328 mg (2 mmol) AIBN und zusätzlich als Kettenübertragungsreagens 20 mmol Butylmercaptan eingesetzt werden. Man erhält das Polymer wie unter B1, jedoch vom Molekulargewicht (GPC; DMF, 85°C) $M_n$ = 12000 und $M_w$ = 15000.

B1c) Eine weitere Polymerisation wird durchgeführt wie unter B1 beschrieben, wobei jedoch auf 60 g (0,2 Mol) des Monomers A1 328 mg (2 mmol) AIBN und zusätzlich als Kettenübertragungsreagens 4 mmol Butylmercaptan eingesetzt werden. Man erhält das Polymer wie unter B1, jedoch vom Molekulargewicht (GPC; DMF, 85°C) $M_n$ = 25000 und $M_w$ = 44000.

B2) Poly[4-(3-Methacryloxy]-2-hydroxypropyl)-1,2,2,6,6-pentamethylpiperidin]

Beispiel B2a: Gemäss B1) werden 60 g (0,2 Mol) A2) mit 624 mg AIBN in 300 ml Toluol polymerisiert, wobei die Umfällung jedoch durch Lösen in THF und erneutes Fällen in n-Hexan vorgenommen wird. Man erhält 53,2 g (89 %) feines Polymerpulver.

| Mikroanalyse: | | |
|---|---|---|
| | berechnet | gefunden |
| C | 65.14 | 64.72 |
| H | 9.97 | 9.91 |
| N | 4.47 | 4.17 |

$^1$H-NMR ($CDCl_3$): Keine Doppelbindungen sichtbar
TGA ($N_2$, 10°C/Min): 5 % Gewichtsverlust bei 320°

GPC (DMF, 85°C): $M_n$: 54000
$M_w$: 158000

Beispiel B2b: Durch Ausführen der obigen (B2a) Vorschrift und Umfällen gemäß Beispiel B1 durch Lösen in Toluol und erneutes Fällen in n-Hexan erhält man ein Polymer mit dem Molekulargewicht (GPC; DMF, 85°C) $M_n$= 93000 und $M_w$= 470000.

B2c) Eine weitere Polymerisation wird durchgeführt wie unter B2a beschrieben, wobei jedoch auf 60 g (0,2 Mol) des Monomers A2 328 mg (2 mmol) AIBN und zusätzlich als Kettenübertragungsreagens 40 mmol Butylmercaptan eingesetzt werden. Man erhält das Polymer wie unter B2a, jedoch vom Molekulargewicht (GPC; DMF, 85°C) $M_n$ = 7800 und $M_w$ = 9200.

B2d) Eine weitere Polymerisation wird durchgeführt wie unter B2a beschrieben, wobei jedoch auf 60 g (0,2 Mol) des Monomers A2 328 mg (2 mmol) AIBN und zusätzlich als Kettenübertragungsreagens 20 mmol Butylmercaptan eingesetzt werden. Man erhält das Polymer wie unter B2a, jedoch vom Molekulargewicht (GPC; DMF, 85°C) $M_n$ = 12000 und $M_w$ = 15000.

B2e) Eine weitere Polymerisation wird durchgeführt wie unter B2a beschrieben, wobei jedoch auf 60 g (0,2 Mol) des Monomers A2 1,6 g (10 mmol) AIBN und zusätzlich als Kettenübertragungsreagens 10 mmol Butylmercaptan eingesetzt werden. Man erhält das Polymer wie unter B2a, jedoch vom Molekulargewicht (GPC; DMF, 85°C) $M_n$ = 15000 und $M_w$ = 21000.

B2f) Eine weitere Polymerisation wird durchgeführt wie unter B2a beschrieben, wobei jedoch auf 60 g (0,2 Mol) des Monomers A2 328 mg (2 mmol) AIBN und zusätzlich als Kettenübertragungsreagens 10 mmol Butylmercaptan eingesetzt werden. Man erhält das Polymer wie unter B2a, jedoch vom Molekulargewicht (GPC; DMF, 85°C) $M_n$ = 17000 und $M_w$ = 24000.

B2g) Eine weitere Polymerisation wird durchgeführt wie unter B2a beschrieben, wobei jedoch auf 60 g (0,2 Mol) des Monomers A2 328 mg (2 mmol) AIBN und zusätzlich als Kettenübertragungsreagens 4 mmol Butylmercaptan eingesetzt werden. Man erhält das Polymer wie unter B2a, jedoch vom Molekulargewicht (GPC; DMF, 85°C) $M_n$ = 25000 und $M_w$ = 42000.

B3) Poly{4[3-(4-Vinylbenzoyloxy)-2-hydroxypropyloxy]-1,2,2,6,6pentamethylpiperidin}

Gemäss B1) werden 40 g (107 mmol) A3) mit 525 mg AIBN in 120 g Toluol polymerisiert.
Man erhält 28,8 g (72 %) weisses Polymer.

| Mikroanalyse: | | |
|---|---|---|
| | berechnet | gefunden |
| C | 70.37 | 69.79 |
| H | 8.86 | 8.74 |
| N | 3.73 | 3.47 |

$^1$H-NMR ($CDCl_3$): Keine Doppelbindungen sichtbar
TGA ($N_2$, 10°C/Min): 10 % Gewichtsverlust bei 360°C

GPC (DMF): $M_n$: 53000
$M_w$: 123000

B3a) Eine weitere Polymerisation wird durchgeführt wie unter B3) beschrieben, wobei jedoch auf 40 g (107 mmol) des Monomers A3 526 mg (ca. 3 mmol) AIBN und zusätzlich als Kettenübertragungsreagens 5 mmol Butylmercaptan eingesetzt werden. Man erhält das Polymer wie unter B3), jedoch vom Molekulargewicht (GPC; DMF, 85°C) $M_n$ = 14000 und $M_w$ = 17000.

B3b) Eine weitere Polymerisation wird durchgeführt wie unter B3) beschrieben, wobei jedoch auf 40 g (107 mmol) des Monomers A3 526 mg (ca. 3 mmol) AIBN und zusätzlich als Kettenübertragungsreagens 10 mmol Butylmercaptan eingesetzt werden. Man erhält das Polymer wie unter B3), jedoch vom Molekulargewicht (GPC; DMF, 85°C) $M_n$ = 11000 und $M_w$ = 12000. Eine Verdoppelung der Menge an AIBN in einer weiteren Präparation führt zu keiner signifikanten Änderung des Molekulargewichts.

B4) Poly{1[3-(Vinylbenzoyloxy)-2-hydroxypropyl]-4-benzyloxy-2,2,6,6-tetra- methylpiperidin}

Gemäss B1) wurden 51 g (113 mmol) A4) mit 557 mg (3,4 mmol) AIBN in 153 ml Toluol bei 70°C polymerisiert.

Nach zweimaligen Umfällen erhält man 45,6 g (89 %) weisses Polymerpulver.

| Mikroanalyse | | |
|---|---|---|
| | berechnet | gefunden |
| C | 74.47 | 74.01 |
| H | 8.26 | 8.22 |
| N | 3.10 | 3.00 |

TGA ($N_2$, 10°C/Min): 10 % Gewichtsverlust bei 320°

GPC (DMF): $M_n$: 52000
$M_w$: 114000

B5) Poly[1-(3-Acryloxy-2-hydroxypropyl)-4-benzyloxy-2,2,6,6-tetramethylpiperidin]

Analog B1) werden 37 g (98,5 mMol) der gemäss Beispiel A5) hergestellten Verbindung mit 485 mg AIBN (3 mMol) in 111 ml Toluol bei 70°C polymerisiert.

Nach zweimaligem Umfällen in THF/n-Hexan erhält man 30,1 g (81 %) eines weissen Polymerpulvers.

| Mikroanalyse: | | | |
|---|---|---|---|
| | % C | % H | % N |
| berechnet | 70,37 | 8,86 | 3,73 |
| gefunden | 69,81 | 9,02 | 3,72 |

TGA ($N_2$, 10°C/Min): 10 % Gewichtsverlust bei 310°C
DSC ($N_2$, 10°C/Min): Schmelzpunkt bei 337°C
GPC (THF): Mn: 31000

Mw: 63000

B6) Poly[1-(3-Methacryloxy-2-hydroxypropyl)4-benzyloxy-2,2,6,6-tetramethylpiperidin]

Analog B1) werden 50 g (128 mMol) der gemäss Beispiel A6 hergestellten Verbindung mit 632 mg (3,9 mMol) AIBN in 150 ml Toluol bei 70°C polymerisiert. Man erhält 41,4 g (82 %) des Titelproduktes als weisses Polymerpulver.

| Mikroanalyse: | | | |
|---|---|---|---|
| | C % | H % | N % |
| berechnet | 70,92 | 9,06 | 3,60 |
| gefunden | 70,38 | 8,95 | 3,61 |

TGA ($N_2$, 10°C/Min): 10 % Gewichtsverlust bei 330°C
DSC ($N_2$, 10°C/Min): 337°C
GPC (THF): Mn: 59000
Mw: 180000

B7) Poly{1-(3-[4-Vinylphenoxy]-2-hydroxypropyl)-4-benzyloxy-2,2,6,6-tetramethyl- piperidin}

In einem 250 ml Rundkolben mit Magnetrührer und Argonballon werden 73 g (172 mMol) des Monomeren aus Beispiel A7, 848 mg (5,2 mMol) AIBN und 146 ml Toluol vorgelegt. Die Lösung wird von Sauerstoff befreit und unter Argon bei 70°C während 20 Stunden gerührt.

Das Polymer wird in n-Hexan gefällt, isoliert und in THF gelöst. Erneut wird in n-Hexan gefällt und das Polymer bei 60°C/0,01 Torr getrocknet.

Man erhält 29 g (40 %) weisses Polymer.

| Mikroanalyse: | | | |
|---|---|---|---|
| | C % | H % | N % |
| berechnet | 76,56 | 8,80 | 3,31 |
| gefunden | 75,94 | 8,77 | 2,67 |

GPC (DMF) Mn: 29000
Mw: 42000
TGA ($N_2$, 10°C/min): 5 % Masseverlust bei 320°C.

B8) Poly{4-(3-[4-Vinylphenoxy]-2-hydroxypropyloxy)-1,2,2,6,6-pentamethylpiperidin}

35 g (101 mMol) der Monomeren aus Beispiel A8 werden mit 496 mg (3 mMol) AIBN in 105 ml Toluol gelöst und der Sauerstoff aus der Lösung entfernt.

Unter Argon lässt man bei 70°C für 16 Stunden rühren. Das Polymer wird dann in n-Hexan ausgefällt, in THF gelöst und erneut in n-Hexan ausgefällt.

Das weisse Polymerpulver wird bei 60°C/0,008 Torr getrocknet.

Ausbeute: 17,4 g (50 %)

| Mikroanalyse: | | | |
|---|---|---|---|
| | C % | H % | N % |
| berechnet | 72,58 | 9,57 | 4,03 |
| gefunden | 72,54 | 9,54 | 3,72 |

GPC (DMF): Mw: 33000
Mn: 23000
TGA ($N_2$, 10°C/min): 5 % Masseverlust bei 380°C.

C) Anwendungsbeispiele

Beispiel C1: Lichtstabilisierung von Polypropylenfasern

Je 2,5 g des erfindungsgemäßen Stabilisators werden zusammen mit 1 g Tris(2,4-di-tert.-butylphenyl)phosphit, 1 g Calcium-Monoethyl-3,5-di-tert.butyl-4-hydroxybenzylphosphonat, 1 g

Calciumstearat und 2,5 g $TiO_2$ (Kronos RN 57) in einem Turbomischer mit 1000 g Polypropylenpulver vermischt (Schmelzindex 12 g/10 min, gemessen bei 230°C/2,16 kg).
Die Mischungen werden bei 200-230°C zu Granulat extrudiert; dieses wird anschließend mit Hilfe einer Pilotanlage (Leonard; Sumirago/VA, Italy) unter folgenden Bedingungen zu Fasern verarbeitet:

| | |
|---|---|
| Extrudertemperatur: | 190-230°C |
| Kopftemperatur: | 255-260°C |
| Streckverhältnis: | 1:3,5 |
| Strecktemperatur: | 100°C |
| Fasern: | 10 den |

Die so hergestellten Fasern werden vor weißem Hintergrund in einem Weather-O-Meter® Typ 65WR (Atlas Corp.) mit einer Schwarztafeltemperatur von 63°C gemäß ASTM D 2565-85 belichtet. Nach unterschiedlichen Belichtungszeiten wird die verbliebene Zugfestigkeit der Proben gemessen. Aus den Meßwerten wird die Belichtungszeit $T_{50}$ berechnet, nach der die Zugfestigkeit der Proben nur noch halb so groß ist.

Zu Vergleichszwecken werden Fasern ohne erfindungsgemäßen Stablisator unter sonst gleichen Bedingungen hergestellt und getestet. Die Testergebnisse sind in Tabelle C1 zusammengestellt.

Tab. C1:

| Belichtungsdauer bis Halbierung der anfänglichen Zugfestigkeit | |
|---|---|
| Stabilisator | Belichtungsdauer |
| keine | 300 h |
| aus Beispiel B1 | 1120 h |
| aus Beispiel B2b | 1030 h |
| aus Beispiel B5 | 1050 h |
| aus Beispiel B6 | 1060 h |
| aus Beispiel B8 | 1180 h |

Die erfindungsgemäß stabilisierten Fasern weisen einen hervorragenden Festigkeitserhalt auf.
Beispiel C2: Stabilisierung eines Zweischicht-Lackes
Die Lichtschutzmittel werden in 5-10 g Xylol eingearbeitet und in einem Klarlack folgender Zusammensetzung geprüft:

| | |
|---|---|
| Synthacryl® SC 303 [1)] | 27,51 |
| Synthacryl® SC 370 [2)] | 23,34 |
| Maprenal® MF 650 [3)] | 27,29 |
| Butylacetat/Butanol (37/8) | 4,33 |
| Isobutanol | 4,87 |
| Solvesso® 150 [4)] | 2,72 |
| Kristallöl K-30 [5)] | 8,74 |
| Verlaufshilfsmittel Baysilon® MA [6)] | 1,20 |
| | 100,00 g |

1) Acrylatharz, Fa. Hoechst AG; 65 % Lösung in Xylol/Butanol 26:9
2) Acrylatharz, Fa. Hoechst AG; 75 % Lösung in Solvesso®100[4)]
3) Melaminharz, Fa. Hoechst AG; 55 % Lösung in Isobutanol
4) Hersteller: Fa. ESSO
5) Hersteller: Fa. Shell
6) 1 % in Solvesso® 150; Hersteller: Fa. Bayer AG

Dem Klarlack werden 1 % Stabilisator zugesetzt, bezogen auf den Feststoffgehalt des Lackes. Als Vergleich dient ein Klarlack, der kein Lichtschutzmittel enthält.

Der Klarlack wird mit Solvesso® 100 auf Spritzfähigkeit verdünnt und auf ein vorbereitetes Aluminiumblech (coil coat, Füller, silbermetallic Basislack) gespritzt und bei 130°C 30 Minuten eingebrannt. Es resultiert eine Trockenfilmdicke von 40-50 μm Klarlack.

Die Proben werden dann in einem UVCON®-Bewitterungsgerät der Fa. Atlas Corp. (UVB-313 Lampen) bei einem Zyklus von 4 h UV-Bestrahlung bei 60°C und 4 h Kondensation bei 50°C bewittert.

Die Proben werden in regelmässigen Abständen auf Risse untersucht. Die Ergebnisse sind in Tabelle C2 aufgeführt.

Tab. C2:

| Bewitterungsdauer bis zur Rissbildung | |
|---|---|
| Stabilisator | Rissbildung nach |
| ohne | 1200 h |
| aus Beispiel B3 | 4000 h |
| aus Beispiel B4 | 4800 h |
| aus Beispiel B5 | 4800 h |
| aus Beispiel B6 | 4800 h |

Die Proben mit den erfindungsgemäßen Stabilisatoren weisen eine hohe Beständigkeit gegenüber Rissbildung auf.

Beispiel C3: Stabilisierung eines photographischen Materials

0,087 g des Gelbkupplers der Formel

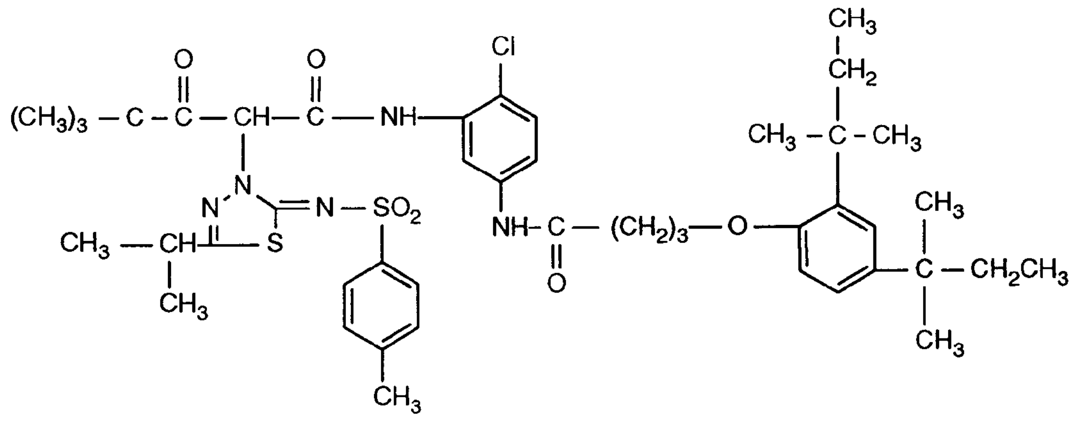

werden in 2,0 ml einer Lösung des erfindungsgemässen Stabilisators in Ethylacetat (2,25 g/100 ml) gelöst. Zu 1,0 ml dieser Lösung gibt man 9,0 ml einer 2,3%-igen wässrigen Gelatinelösung, die auf einem pH-Wert von 6,5 eingestellt ist, und 1,744 g/l des Netzmittels der Formel

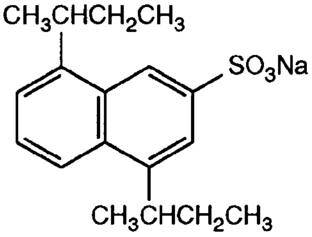

enthält.

Zu 5,0 ml der so erhaltenen Kuppleremulsion gibt man 2 ml einer Silberbromid-Emulsion mit einem Silbergehalt von 6,0 g/l sowie 1,0 ml einer 0,7%-igen wässrigen Lösung des Härters der Formel

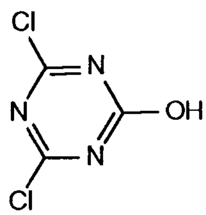

und vergiesst es auf ein 13 x 18 cm Kunststoff-beschichtetes Papier. Nach einer Härtungszeit von 7 Tagen werden die Proben hinter einem Silber-Stufenkeil mit 125 Lux·s belichtet und anschliessend im Ektaprint 2®-Prozess der Firma Kodak verarbeitet.

Die erhaltenen Gelbkeile werden in einem Atlas Weather-Ometer mit einer 2500 W-Xenonlampe hinter einem UV-Filter (Kodak 2C) mit total 60 k Joule/cm$^2$ bestrahlt.

Eine Probe ohne Stabilisator läuft als Standard mit.

Der bei der Bestrahlung eingetretene Farbdichteverlust beim Absorptionsmaximum des gelben Farbstoffes wird mit einem Densitometer TR 924A der Fa. Macbeth gemessen.

Der Lichtschutzeffekt ist aus dem Farbdichteverlust ersichtlich. Je kleiner der Dichteverlust, desto höher ist die Lichtschutzwirksamkeit.

Die erfindungsgemässen Stabilisatoren zeigen eine gute Lichtschutzwirkung.

Beispiel C4: Stabilisierung von Polypropylen-Bändern

1,0 g des erfindungsgemäßen Stabilisators werden zusammen mit 1 g Tris(2,4-di-tert.-butylphenyl)phosphit, 0,5 g Pentaerythrityl-tetrakis(3-[3',5'-di-tert.butyl-4'-hydroxyphenyl]-propionat) und 1 g Calciumstearat in einem Turbomischer mit 1000 g Polypropylenpulver vermischt (STATOIL MF; Schmelzindex 4,0 g/10 min, gemessen bei 230°C/2,16 kg).

Die Mischungen werden bei 200-230°C zu Granulat extrudiert; dieses wird anschließend mit Hilfe einer Pilotanlage (Leonard; Sumirago/VA, Italy) unter folgenden Bedingungen zu 2,5 mm breiten

Streckbändern von 50 µm Dicke verarbeitet:

| | |
|---|---|
| Extrudertemperatur: | 210-230°C |
| Kopftemperatur: | 240-260°C |
| Streckverhältnis: | 1:6 |
| Strecktemperatur: | 110°C |

Die so hergestellten Fasern werden vor weißem Hintergrund in einem Weather-O-Meter® Typ 65WR (Atlas Corp.) mit einer Schwarztafeltemperatur von 63°C gemäß ASTM D 2565-85 belichtet. Nach unterschiedlichen Belichtungszeiten wird die verbliebene Zugfestigkeit der Proben gemessen. Aus den Meßwerten wird die Belichtungszeit $T_{50}$ berechnet, nach der die Zugfestigkeit der Proben nur noch halb so groß ist.

Zu Vergleichszwecken werden Fasern ohne erfindungsgemäßen Stablisator unter sonst gleichen Bedingungen hergestellt und getestet. Die Testergebnisse sind in Tabelle C4 zusammengestellt.

Tab. C4:

| Belichtungsdauer bis Halbierung der anfänglichen Zugfestigkeit | |
|---|---|
| Stabilisator | Belichtungsdauer |
| keiner | 360 h |
| aus Beispiel B1 | 1220 h |

Die erfindungsgemäß stabilisierte Probe weist einen hervorragenden Festigkeitserhalt auf.

## Patentansprüche

1. Ein Polymer mit Tetramethylpiperidin-Seitenketten enthaltend 50 bis 100 Mol-% strukturelle Einheiten der Formel I und/oder II

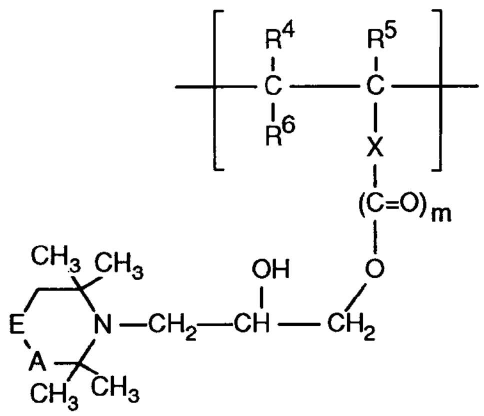

(I)

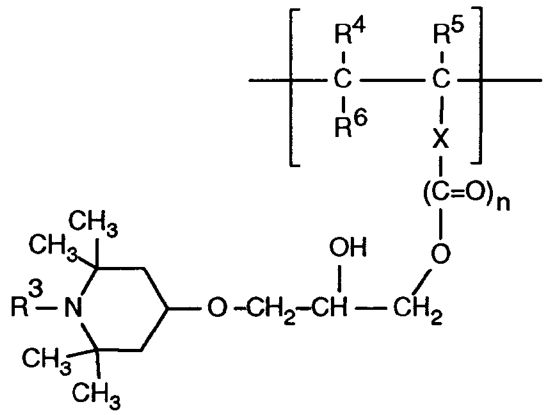

(II)

wobei das mittels Gelpermeationschromatographie gemessene Molekulargewicht $M_n$ des Homo- oder Copolymers zwischen 600 und 600 000 g/Mol beträgt, und wobei das unten definierte k die Zahl 2 oder 3 ist;
m und n, unabhängig voneinander, 0 oder 1 darstellen;
A $-CH_2-$ oder -CO- bedeutet;
wenn A Methylen ist, E die Bedeutung

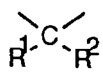

hat, und
wenn A Carbonyl ist, E die Bedeutung $>N-R^{17}$ hat;
$R^1$ Wasserstoff;
$R^2$ Wasserstoff; $-N(R^{13})R^{14}$; oder $C_1$-$C_{50}$-Alkoxy; oder $C_2$-$C_{50}$-Alkoxy bedeutet, welches durch -O-, -S-, $-CO-N(R^{17})-$, $-N(R^{17})-CO-$ oder $-NR^{11}-$ und/oder durch $C_5$-$C_8$-Cycloalkylen oder Phenylen unterbrochen ist und/oder welches 1 bis 3 tertiäre Hydroxylgruppen enthält; oder $R^2$ $C_5$-$C_{12}$-Cycloalkoxy; durch 1 bis 4 Reste $-R^{12}$ substituiertes $C_5$-$C_{12}$-Cycloalkoxy; Phenoxy; durch 1 bis 4 $-R^{12}$ substituiertes Phenoxy; oder $C_7$-$C_{20}$-Aralkoxy bedeutet; oder
$R^1$ und $R^2$ gemeinsam einen Substituenten =O bedeuten; oder zusammen mit dem Kohlenstoffatom, an das sie gebunden sind, einen Fünf- oder Sechsring einer der Formeln

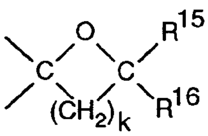, 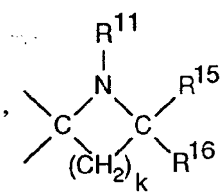, 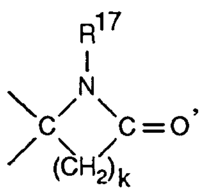, 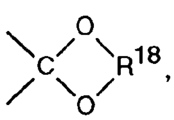, 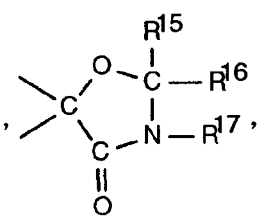,

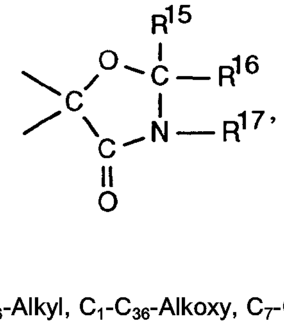, 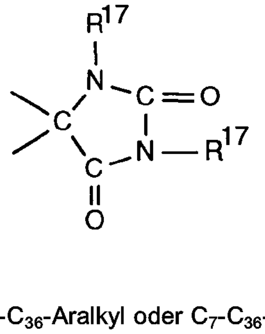

darstellen;
$R^3$ Wasserstoff; $C_1$-$C_{36}$-Alkyl, $C_1$-$C_{36}$-Alkoxy, $C_7$-$C_{36}$-Aralkyl oder $C_7$-$C_{36}$-Aralkoxy, die jeweils unsubstituiert oder durch $C_5$-$C_8$-Cycloalkyl oder $-CO-N(R^{17})_2$ substituiert oder im aliphatischen Teil durch $C_5$-$C_8$-Cycloalkylen $-CO-N(R^{17})-$ oder $-N(R^{17})-CO-$ oder Sauerstoff oder Schwefel unterbrochen oder im aromatischen Teil durch 1 bis 3 $C_1$-$C_4$-Alkyl- oder $C_1$-$C_4$-Alkoxyreste substituiert sind; $C_5$-$C_{12}$-Cycloalkyl, welches unsubstituiert oder durch 1 bis 3 $C_1$-$C_4$-Alkyl- oder $C_1$-$C_4$-Alkoxyreste substituiert ist; $C_5$-$C_{12}$-Cycloalkoxy, welches unsubstituiert oder durch 1 bis 3 $C_1$-$C_4$-Alkyl- oder $C_1$-$C_4$-Alkoxyreste substituiert ist; $C_6$-$C_{10}$-Aryl, welches unsubstituiert oder durch 1 bis 3 $C_1$-$C_4$-Alkyl- oder $C_1$-$C_4$-Alkoxyreste substituiert ist; $C_6$-$C_{10}$-Aryloxy, welches unsubstituiert oder durch 1 bis 3 $C_1$-$C_4$-Alkyl- oder $C_1$-$C_4$-Alkoxyreste substituiert ist; -CO-$R^{11}$; oder Benzoyl oder Naphtoyl, welche jeweils durch $C_1$-$C_4$-Alkyl substituiert sind, bedeutet;
$R^4$ und $R^5$, unabhängig voneinander, Wasserstoff; $C_1$-$C_{18}$-Alkyl; Phenyl; oder $C_7$-$C_9$-Phenylalkyl sind; oder einer der $R^4$ oder $R^5$ zusätzlich Chlor umfaßt;
$R^6$ eine der für $R^4$ und $R^5$ angegebenen Bedeutungen außer Chlor hat; oder eine direkte Bindung zur Gruppe X bedeutet; oder eine Gruppe einer der Formeln -X-$(CO)_m$-$Z^4$ oder -X-$(CO)_n$-$Z^5$ darstellt;
$R^{11}$ $C_1$-$C_{18}$-Alkyl; $C_5$-$C_7$-Cycloalkyl; Phenyl; Naphthyl; $C_7$-$C_9$-Phenylalkyl; oder $C_{11}$-$C_{14}$-Naphthylalkyl;
$R^{12}$ $C_1$-$C_{18}$-Alkyl; $C_5$-$C_7$-Cycloalkyl; Phenyl oder Benzyl ist;
$R^{13}$ und $R^{14}$, unabhängig voneinander, $C_1$-$C_{50}$-Alkyl; oder $C_2$-$C_{50}$-Alkyl bedeuten, welches durch -O-, -S-, -CO-N($R^{17}$)-, -N($R^{17}$)-CO- oder -$NR^{11}$- und/oder durch $C_5$-$C_8$-Cycloalkylen oder Phenylen unterbrochen ist und/oder welches 1 bis 3 tertiäre Hydroxylgruppen enthält; oder $C_5$-$C_{12}$-Cycloalkyl, welches unsubstituiert oder durch 1 bis 4 Reste -$R^{12}$ substituiert ist; Phenyl, welches unsubstituiert oder durch 1 bis 4 -$R^{12}$ substituiert ist; oder $C_7$-$C_{20}$-Aralkyl bedeuten; oder
$R^{13}$ und $R^{14}$ zusammen mit dem Stickstoffatom, an das sie gebunden sind, ein zyklisches Imid der Formel

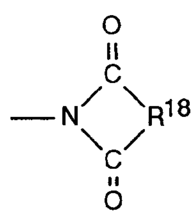

darstellen, dessen Ringstruktur 4 bis 6 Kohlenstoffatome enthält;
$R^{15}$ und $R^{16}$, unabhängig voneinander, H; oder $C_1$-$C_{12}$-Alkyl; oder gemeinsam geradkettiges, $\alpha,\omega$-verknüpftes $C_4$-$C_{13}$-Alkylen bedeuten;
$R^{17}$ Wasserstoff ist oder eine der Bedeutungen von $R^{11}$ hat;
$R^{18}$ $C_2$-$C_{18}$-Alkylen darstellt;
X im Fall, daß $R^6$ eine direkte Bindung zur Gruppe X darstellt, die trivalente Gruppe

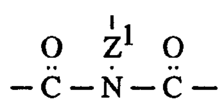

ist, wobei die an den beiden Carbonylgruppen lokalisierten freien Valenzen an die benachbarten Kohlenstoffatome der Polymerkette binden;
und X, wenn $R^6$ keine direkte Bindung zur Gruppe X darstellt, im Fall m = 0 beziehungsweise n = 0 Phenylen oder durch -$Z^4$ oder -$Z^5$ substituiertes Phenylen ist; und X, wenn $R^6$ keine direkte Bindung zur Gruppe X darstellt, im Fall m = 1 beziehungsweise n = 1 eine direkte Bindung; $C_1$-$C_4$-Alkylen; Phenylen; oder -$Z^2$-$Z^3$- bedeutet, wobei $Z^2$ an das Kohlenstoffatom der Polymerkette bindet;
$Z^1$ Phenylen;
$Z^2$-O- oder Phenylen;
$Z^3$ $C_1$-$C_8$-Alkylen;
$Z^4$ eine Gruppe der Formel Ia

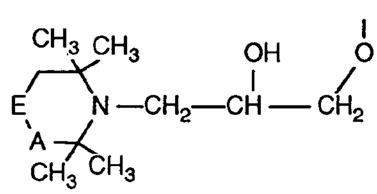

(Ia)

und $Z^5$ eine Gruppe der Formel IIa

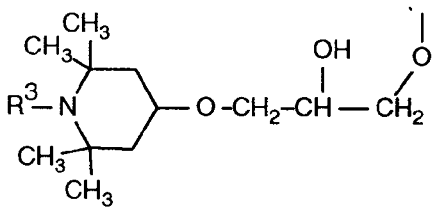

(IIa)

darstellt.

2. Homo- oder Copolymer gemäß Anspruch 1, enthaltend 50 bis 100 Mol-% Struktureinheiten der Formel I und/oder II, sowie 0 bis 50 Mol-% Struktureinheiten der Formel III

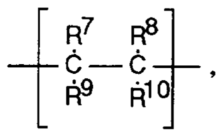

(III)

dessen mittels Gelpermeationschromatographie gemessenes Molekulargewicht $M_n$ zwischen 1000 und 300 000 g/Mol beträgt, und worin
$R^7$, $R^8$ und $R^9$, unabhängig voneinander, Wasserstoff; -Cl; $C_1$-$C_{18}$-Alkyl; Phenyl; Phenyl, welches durch 1 bis 3 -Cl, $C_1$-$C_4$-Alkyl und/oder $C_1$-$C_4$-Alkoxy substituiert ist; oder $C_7$-$C_9$-Phenylalkyl sind;
$R^{10}$ eine der für $R^7$, $R^8$ und $R^9$ angegebenen Bedeutungen hat; oder -CN; $C_1$-$C_{12}$-Alkyloxycarbonyl; $C_1$-$C_{12}$-Alkanoyloxy; $C_1$-$C_{12}$-Alkoxy; oder eine Gruppe der Formel IVa oder IVb ist

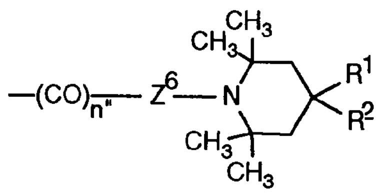

(IVa)

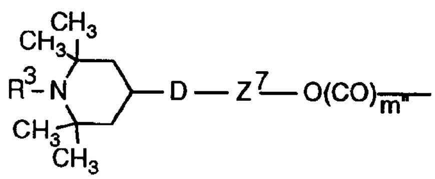

(IVb)

worin m" und n" jeweils die Zahl 0 oder die Zahl 1 darstellen;
D -O- oder -$NR^{11}$- ist;
$R^1$, $R^2$, $R^3$ und $R^{11}$ die in Anspruch 1 angegebenen Bedeutungen haben;
$Z^6$ eine direkte Bindung, $C_1$-$C_8$-Alkylen oder Polyoxyethylen gemäß der Formel $-CH_2-CH_2-(O-CH_2-CH_2)_h-$ darstellt, wobei h eine ganze Zahl aus dem Bereich 1 bis 30 ist; und
$Z^7$ $C_1$-$C_8$-Alkylen oder Polyoxyethylen gemäß der Formel $-CH_2-CH_2-(O-CH_2-CH_2)_h-$ darstellt, wobei h eine ganze Zahl aus dem Bereich 1 bis 30 ist.

3. Homo- oder Copolymer gemäß Anspruch 2, wobei das Molekulargewicht $M_n$ des Homopolymeren aus Struktureinheiten der Formel I 1000 bis 300 000 g/Mol, das Molekulargewicht $M_n$ des Homopolymeren aus Struktureinheiten der Formel II 1000 bis 30 000 g/Mol und das Molekulargewicht $M_n$ des Copolymeren 1000 bis 100 000 g/Mol beträgt.

4. Homo- oder Copolymer gemäß Anspruch 1, enthaltend 50 bis 100 Mol-% Struktureinheiten der Formel I und/oder II, worin
A Methylen und E eine Gruppe der Formel

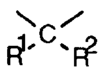

ist;

$R^1$ Wasserstoff;

$R^2$ Wasserstoff; $-N(R^{13})R^{14}$; $C_1$-$C_{18}$-Alkoxy; $C_4$-$C_{36}$-Alkoxy, welches durch $-CO-N(R^{17})-$ oder $-N(R^{17})-CO-$ unterbrochen ist; $C_5$-$C_8$-Cycloalkoxy, welches unsubstituiert oder durch $-R^{12}$ substituiert ist; Phenoxy, welches unsubstituiert oder durch 1 bis 4 $-R^{12}$ substituiert ist; oder $C_7$-$C_{12}$-Aralkoxy bedeutet; oder

$R^1$ und $R^2$ gemeinsam einen Substituenten =O bedeuten oder zusammen mit dem Kohlenstoffatom, an das sie gebunden sind, einen Fünf- oder Sechsring einer der Formeln

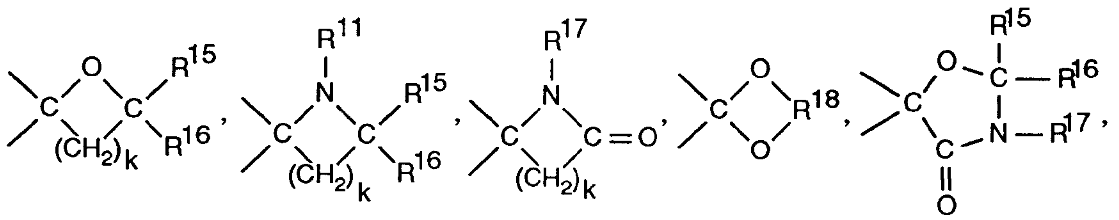

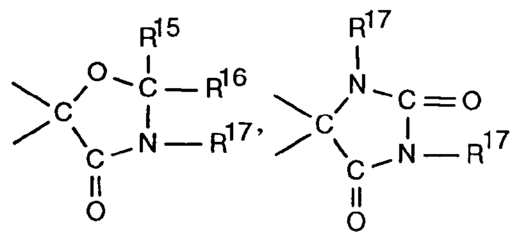

darstellen;

$R^3$ die Bedeutung $C_1$-$C_{36}$-Alkyl; $C_1$-$C_{36}$-Alkoxy; $C_5$-$C_{12}$-Cycloalkyl, welches unsubstituiert oder durch 1 bis 3 $C_1$-$C_4$-Alkyl- oder $C_1$-$C_4$-Alkoxyreste substituiert ist; $C_5$-$C_{12}$-Cycloalkoxy, welches unsubstituiert oder durch 1 bis 3 $C_1$-$C_4$-Alkyl- oder $C_1$-$C_4$-Alkoxyreste substituiert ist; Phenyl, welches unsubstituiert oder durch 1 bis 3 $C_1$-$C_4$-Alkyl- oder $C_1$-$C_4$-Alkoxyreste substituiert ist; Naphthyl, welches unsubstituiert oder durch 1 bis 3 $C_1$-$C_4$-Alkyl- oder $C_1$-$C_4$-Alkoxyreste substituiert ist; Phenoxy, welches unsubstituiert oder durch 1 bis 3 $C_1$-$C_4$-Alkyl- oder $C_1$-$C_4$-Alkoxyreste substituiert ist; Naphthoxy, welches unsubstituiert oder durch 1 bis 3 $C_1$-$C_4$-Alkyl- oder $C_1$-$C_4$-Alkoxyreste substituiert ist; $C_7$-$C_{12}$-Phenylalkyl, welches unsubstituiert oder am Phenylring durch 1 bis 3 $C_1$-$C_4$-Alkyl- oder $C_1$-$C_4$-Alkoxyreste substituiert ist; $C_7$-$C_{12}$-Phenylalkoxy, welches unsubstituiert oder am Phenylring durch 1 bis 3 $C_1$-$C_4$-Alkyl- oder $C_1$-$C_4$-Alkoxyreste substituiert ist; $-CO-R^{11}$; oder Benzoyl oder Naphtoyl, welche jeweils durch $C_1$-$C_4$-Alkyl substituiert sind, hat;

$R^4$, $R^5$ und $R^6$, unabhängig voneinander, Wasserstoff; $C_1$-$C_{18}$-Alkyl; Phenyl; oder $C_7$-$C_9$-Phenylalkyl sind; oder einer der $R^4$ oder $R^5$ zusätzlich -Cl umfaßt;

$R^{11}$ $C_1$-$C_{18}$-Alkyl; $C_5$-$C_7$-Cycloalkyl; Phenyl; oder $C_7$-$C_9$-Phenylalkyl;

$R^{12}$ $C_1$-$C_{18}$-Alkyl; $C_5$-$C_7$-Cycloalkyl; Phenyl oder Benzyl ist;

$R^{13}$ und $R^{14}$, unabhängig voneinander, $C_1$-$C_{18}$-Alkyl; $C_4$-$C_{36}$-Alkyl, welches durch $-CO-N(R^{17})-$ oder $-N(R^{17})-CO-$ unterbrochen ist; $C_5$-$C_8$-Cycloalkyl, welches unsubstituiert oder durch $-R^{12}$ substituiert ist; Phenyl, welches unsubstituiert oder durch 1 bis 4 $-R^{12}$ substituiert ist; oder $C_7$-$C_{12}$-Aralkyl bedeutet;

$R^{15}$ und $R^{16}$, unabhängig voneinander, H; oder $C_1$-$C_{12}$-Alkyl; oder gemeinsam geradkettiges, $\alpha,\omega$-verknüpftes $C_4$-$C_{13}$-Alkylen bedeuten;

$R^{17}$ Wasserstoff ist oder eine der Bedeutungen von $R^{11}$ hat;

X im Fall m = 0 beziehungsweise n = 0 Phenylen oder in 5-Stellung durch $-Z^4$ oder $-Z^5$ substituiertes 1,3-Phenylen ist;

X im Fall m = 1 beziehungsweise n = 1 eine direkte Bindung; $C_1$-$C_4$-Alkylen; Phenylen; oder $-Z^2-Z^3-$ bedeutet, wobei $Z^2$ an das Kohlenstoffatom der Polymerkette bindet.

5. Homo- oder Copolymer gemäß Anspruch 4 enthaltend 50 bis 100 Mol-% Struktureinheiten der Formel I und/oder II, worin

A Methylen und E eine Gruppe der Formel

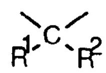

ist;
$R^2$ $-N(R^{13})R^{14}$; oder $C_2$-$C_{18}$-Alkoxy; $C_4$-$C_{36}$-Alkoxy, welches durch $-CO-N(R^{17})-$ oder $-N(R^{17})-CO-$ unterbrochen ist; oder $C_7$-$C_{12}$-Aralkoxy bedeutet; oder $R^1$ und $R^2$ gemeinsam einen Substituenten =O bedeuten;
$R^3$ die Bedeutung $C_1$-$C_{12}$-Alkyl; $C_4$-$C_{18}$-Alkoxy; $C_5$-$C_8$-Cycloalkyl; $C_5$-$C_8$-Cycloalkoxy; $C_7$-$C_{12}$-Phenylalkyl; $C_7$-$C_{12}$-Phenylalkoxy; oder $-CO-R^{11}$ hat;
zwei der Reste $R^4$, $R^5$ und $R^6$ Wasserstoff bedeuten und einer die Bedeutung Wasserstoff; $C_1$-$C_4$-Alkyl; Phenyl; oder $C_7$-$C_9$-Phenylalkyl hat;
$R^{11}$ $C_1$-$C_{18}$-Alkyl; Cyclohexyl; Phenyl; oder $C_7$-$C_9$-Phenylalkyl;
$R^{13}$ und $R^{14}$, unabhängig voneinander, $C_2$-$C_{18}$-Alkyl; $C_4$-$C_{36}$-Alkyl, welches durch $-CO-N(R^{17})-$ oder $-N(R^{17})-CO-$ unterbrochen ist; oder $C_7$-$C_{12}$-Aralkyl bedeuten;
$R^{17}$ Wasserstoff ist oder eine der Bedeutungen von $R^{11}$ hat;
X im Fall m = 0 beziehungsweise n = 0 Phenylen; und
X im Fall m = 1 beziehungsweise n = 1 eine direkte Bindung; $C_1$-$C_4$-Alkylen oder Phenylen darstellt.

**6.** Homo- oder Copolymer gemäß Anspruch 5 enthaltend 50 bis 100 Mol-% Struktureinheiten der Formel I und/oder II, worin
A Methylen und E eine Gruppe der Formel

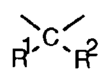

ist;
$R^2$ $C_4$-$C_{18}$-Alkoxy; $C_4$-$C_{36}$-Alkoxy, welches durch $-CO-N(R^{17})-$ oder $-N(R^{17})-CO-$ unterbrochen ist; oder $C_7$-$C_{12}$-Aralkoxy bedeutet; oder $-N(R^{13})R^{14}$ ist; oder $R^1$ und $R^2$ gemeinsam einen Substituenten =O bedeuten;
$R^3$ die Bedeutung $C_1$-$C_6$-Alkyl; $C_4$-$C_{12}$-Alkoxy; Cyclohexyl; Cyclohexyloxy; Benzyl; Benzyloxy; oder Benzoyl hat;
zwei der Reste $R^4$, $R^5$ und $R^6$ Wasserstoff bedeuten und einer die Bedeutung Wasserstoff; Methyl; oder Phenyl hat;
$R^{13}$ und $R^{14}$, unabhängig voneinander, $C_2$-$C_{12}$-Alkyl darstellen;
X im Fall m = 0 beziehungsweise n = 0 Phenylen; und
X im Fall m = 1 beziehungsweise n = 1 eine direkte Bindung; oder Phenylen darstellt.

**7.** Homopolymer gemäß Anspruch 6.

**8.** Verbindung der Formel V

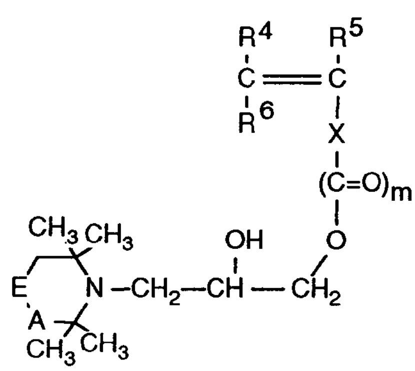

(V)

worin E, A, $R^4$, $R^5$, $R^6$, m und X die in Anspruch 1 angegebenen Bedeutungen haben.

9. Zusammensetzung enthaltend (a) ein gegen Schädigung durch Licht, Sauerstoff und/oder Wärme empfindliches organisches Polymer und (b) als Stabilisator ein Polymer enthaltend strukturelle Einheiten der Formel I und/oder II gemäß Anspruch 1.

10. Zusammensetzung gemäß Anspruch 9, worin Komponente (a) ein Polyolefin oder ein Lackbindemittel auf der Basis von Acryl-, Alkyd-, Polyurethan-, Polyester- oder Polyamidharz oder entsprechender modifizierter Harze ist.

11. Zusammensetzung gemäß Anspruch 9, enthaltend zusätzlich zu den Komponenten (a) und (b) ein konventionelles Additiv.

12. Zusammensetzung gemäß Anspruch 9 enthaltend 0,01 bis 10 Gew.-% der Komponente (b), bezogen auf das Gewicht der Zusammensetzung.

13. Verfahren zum Stabilisieren organischer Polymere gegen Schädigung durch Licht, Sauerstoff und/oder Wärme, dadurch gekennzeichnet, daß man den Polymeren als Stabilisator ein Polymer oder Copolymer gemäß Anspruch 1 beimischt.

14. Verwendung von Polymeren oder Copolymeren gemäß Anspruch 1 zum Stabilisieren von organischen Polymeren gegen Schädigung durch Licht, Sauerstoff und/oder Wärme.

15. Ein gegen schädigenden Einfluß von Licht, Sauerstoff und/oder Wärme geschütztes Polymer, dadurch gekennzeichnet, daß es 0,01 bis 5 Mol-% strukturelle Einheiten der Formel I und/oder II gemäß Anspruch 1 enthält.

16. Ein Pfropfcopolymer enthaltend 2 bis 20 Mol-% strukturelle Einheiten der Formel I und/oder II gemäß Anspruch 1.

17. Verfahren zur Herstellung eines Polymeren gemäß Anspruch 1, dadurch gekennzeichnet, daß man eine Verbindung der Formel V gemäß Anspruch 8 oder eine Verbindung der Formel VI

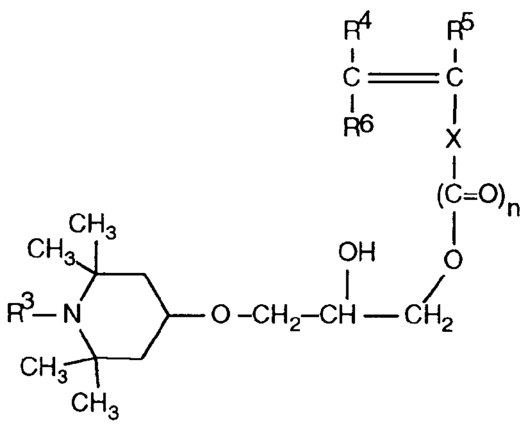

(VI)

worin $R^3$, $R^4$, $R^5$, $R^6$, n und X die in Anspruch 1 angegebenen Bedeutungen haben, oder ein Gemisch aus Verbindungen der Formeln V und VI
oder eine oder mehrere Verbindungen der Formeln V und VI zusammen mit bis zu 50 Mol-% einer Verbindung der Formel VIII

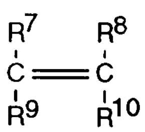

(VIII)

worin $R^7$, $R^8$, $R^9$ und $R^{10}$ die in Anspruch 2 angegebenen Bedeutungen haben, radikalisch polymerisiert.

Europäisches Patentamt

# EUROPÄISCHER RECHERCHENBERICHT

Nummer der Anmeldung: EP 94810398.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl6) |
|---|---|---|---|
| X | EP - A - 0 296 391 (CIBA-GEIGY AG) * Ansprüche 1-6,19,20,23,24; Seite 4, Zeilen 3-35; Seite 5, Zeilen 9-30; Seite 6, Zeilen 15-25; Seite 7, Zeile 31; Seite 9, Zeilen 40-50; Seite 10, Zeilen 15-40 * -- | 1,2, 4-7, 9-11, 13-16 | C 08 F 20/36<br>C 08 F 22/22<br>C 08 F 22/40<br>C 08 F 12/22<br>C 07 D 211/06<br>C 07 D 211/76<br>C 07 D 241/08<br>C 07 D 471/10<br>C 07 D 491/10<br>C 07 D 498/10<br>C 09 D 133/04<br>C 09 D 135/02<br>C 09 D 125/18<br>C 08 K 5/34<br>C 08 L 101/00 |
| X | EP - A - 0 226 538 (CIBA-GEIGY AG) * Ansprüche 1-6,12,13,18,19, 24,25; Seite 2, Zeilen 53-55; Seite 3, Zeilen 2-10; Seite 5, Zeilen 5-15; Seite 8, Zeilen 1-10,29-55 * -- | 1,2, 4-7, 9-11, 13-16 | |
| X | EP - A - 0 063 544 (CIBA-GEIGY AG) * Anspruch 1 mit den Strukturen IV und V; Ansprüche 2-6,11-19 * -- | 1,2, 4-6,9, 13-16 | **RECHERCHIERTE SACHGEBIETE (Int Cl6)** |
| X | EP - A - 0 000 496 (CIBA-GEIGY AG) * Anspruch 1 mit den Strukturen IV und V; Ansprüche 2-6; Zusammenfassung * ---- | 1,2, 4-6,7, 9,13 | C 07 D<br>C 08 F<br>C 08 K<br>C 08 L<br>C 09 D |

Der vorliegende Recherchenbericht wurde für alle Patentansprüche erstellt.

| Recherchenort | Abschlußdatum der Recherche | Prüfer |
|---|---|---|
| WIEN | 21-10-1994 | PUSTERER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82